(19) Europäisches Patentamt / European Patent Office / Office européen des brevets

(11) **EP 3 303 615 B1**

(12) **EUROPEAN PATENT SPECIFICATION**

(45) Date of publication and mention
of the grant of the patent:
**18.09.2019 Bulletin 2019/38**

(51) Int Cl.:
***C12Q 1/6841*** *(2018.01)*

(21) Application number: **16726633.7**

(22) Date of filing: **26.05.2016**

(86) International application number:
**PCT/GB2016/051531**

(87) International publication number:
**WO 2016/193676 (08.12.2016 Gazette 2016/49)**

(54) **HYBRIDISATION METHOD USING IMPROVED NUCLEIC ACID PROBES**

HYBRIDISIERUNGSVERFAHREN MIT VERBESSERTEN NUKLEINSÄURESONDEN

PROCEDE D'HYBRIDATION NECESSITANT DES SONDES D'ACIDE NUCLEIQUE AMELIOREES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.06.2015 GB 201509395**

(43) Date of publication of application:
**11.04.2018 Bulletin 2018/15**

(73) Proprietor: **University of Leicester
Leicester LE1 7RH (GB)**

(72) Inventors:
• **CAUSEY-FREEMAN, Peter
Leicester LE1 7RH (GB)**
• **BROOKES, Anthony
Leicester LE1 7RH (GB)**

(74) Representative: **Wilson Gunn
Charles House
148/9 Great Charles Street
Birmingham B3 3HT (GB)**

(56) References cited:
**WO-A1-2010/040124        US-A- 5 447 841
US-A- 5 985 549              US-A1- 2002 009 728
US-A1- 2013 123 123**

**Description**

Technical field of the invention

This invention relates to the use of probes for the processing of nucleic acid regions of interest (ROIs), and to methods of probe hybridisation and repetitive sequence blocking with non-deoxy nucleic acid sequences, or their synthetic, non-natural equivalents. The various aspects of the invention increase the relative fidelity and effectiveness of probe hybridisation to nucleic acid ROIs to which they were designed to hybridise, versus other hybridisation events.

Background to the invention

For several decades, nucleotide sequences covalently attached to detectable chemistries (probes) have been used to hybridise to and detect or enrich regions of interest (ROIs) comprising nucleic acid sequences within the genomes and transcriptomes of numerous species. Probes of varying sizes have been used for numerous applications ranging from short synthetic oligonucleotides to detect single nucleotide changes in a single ROI, to whole genomes allowing analysis of structural variation between genomes. The techniques can also be applied such that nucleotide sequences are covalently attached to surfaces (surface probes), e.g. microarrays or beads, and the ROI DNA/RNA itself is labelled with a detectable chemistry.

Modern nucleotide and structural analyses often use next generation sequencing (NGS). NGS platforms process immense numbers of DNA fragments, resulting in extremely low cost per base of sequence. Nevertheless, current whole genome sequencing (WGS) performed in ways that ensure that most bases are sequenced a sufficient number of times to permit accurate analysis, costs in excess of £1000 per genome merely to generate the raw data. WGS also outputs vast amounts of sequence requiring storage and expert analysis, so it is not yet feasible to routinely sequence complex genomes in their entirety. This is particularly true in many healthcare and research applications where ROIs comprise only a few genes and WGS yields vast excesses of other genomic sequences. This also presents ethical considerations, such as whether the additional data should be stored and analysed outside of the remit of the investigation and whether the result of such analyses should be disclosed to the patient.

Targeting NGS towards only ROIs reduces the requirement for resources compared to WGS. A number of methods have been developed to recover ROIs, one of which is hybridisation target enrichment (hTE). hTE utilises nucleotide sequences (i.e. probes), or synthetic non-naturally occurring equivalents, attached to recoverable rather than detectable chemistries (recoverable probes are also referred to as 'baits'). The recoverable probes preferentially hybridise to the ROIs and allow physical enrichment of the ROIs over other genomic regions. Elution from the recoverable probe results in a useful degree of purification of the ROIs. hTE can be used for many applications, but is commonly used to enrich ROIs prior to NGS making it more practical and affordable to sequence large numbers of samples, expanding the use of NGS to many more settings.

The cost effectiveness of the leading hTE technologies is good for ROIs greater than at least a few Mbp. Many hTE kits are optimised to recover whole exomes, and this increases associated costs and requirement for resources when only a subset of genes are the focus of the study (though the cost is still significantly less than for WGS). Various reasons exist for the tendency of these kits to target enrichment of exomes or specific gene panels - such as the fact that when targeting larger ROIs poorly effective enrichment methods can still generate a product wherein the majority of the recovered DNA comes from the ROI rather than other genomic regions. The innate 'Enrichment Power' of a method is therefore important.

The Enrichment power (EP or EF) of a method is its efficiency at recovering the targeted ROI compared to its efficiency at recovering other genome regions, and it is calculated as:

$$\frac{\text{The fraction of resulting NGS sequences that overlap the ROI (fr)}}{\text{The fraction of the genome covered by the ROI (ft)}}$$

Considering, for example, the alternatives of targeting ROIs comprising a whole exome (~60 Mb), or a 3 Mb region, or a 300 kb region in the 3,000 Mb human genome, with a requirement that at least 80% of the final sequences from NGS overlap the ROI. These three scenarios would require EPs of at least 40, 800 and 8000 respectively, for a method to be suitable. An EP of ~8000 is unachievable using currently available products. Hence, enrichments of ROIs smaller than many hundred kb are more suited to approaches with vastly superior levels of enrichment specificity, e.g. PCR based procedures (though for larger ROIs, PCR based approaches become impractical). But if targeting a 3 Mb ROI, the required EP of ~800 falls within the top end range of current products. The whole exome enrichment, requiring an EP of ~40, is easily achievable using current products.

While hTE is attractive when fully optimised for a particular ROI (or handful of ROIs), e.g. in a diagnostic setting, applying the same protocol to many different patients without prior optimisation typically leads to unpredictable EP and inconsistent read depth for each ROI base when NGS sequences are aligned to the ROI sequence. This is because genome sequence characteristics vary considerably from region to region, meaning that different ROIs will often have very different repeat sequence and base composition content which require different enrichment reaction conditions.

Regions of genomic DNA (gDNA) sequence containing a high (>70%) GC content tend to denature inefficiently even at very high temperatures. This is further confounded by rapid re-annealing of any fragments that are not fully denatured, once the temperature is subsequently reduced, resulting in these regions exhibiting poor accessibility to probe and poor recovery. In contrast, regions with a low (<30%) GC content tend to denature rapidly but hybridise poorly to probes, again leading to poor recovery. This also affects ROI detection with surface probes reliant on single stringency hybridisation and washing conditions. Since the extreme variability of GC content throughout the genome makes it unlikely that a single set of conditions will be suitable for every surface probe on a microarray, the consequential non-specific hybridisation of some sequences under any one set of conditions has contributed to a reduction in the popularity of microarray based approaches.

Complex genomes typically contain many sequences that are highly similar or identical to sequences at other places in the genome. These 'repeat sequences' comprise well over 60% of the human genome and the majority of exons in the human genome are within a few hundred bp of repeat sequence, or may even have repeat sequence within them. Repeat sequences represent challenges to methods based upon hybridisation because of cross-hybridisation between repeats in ROIs and similar non-ROI copies of those repeats, even under high stringency conditions. This can result in the formation of networks of many ROI and non-ROI DNA fragments that include repeat sequences, leading to: a) poor specificity when using probes to detect an ROI: and b) recovery of genomic regions from outside an ROI, resulting in reduced EP, when performing hTE.

Hybridisation based approaches rarely rely solely on the use of stringent conditions (e.g. high temperatures and low salt concentrations) to favour preferential hybridisation of probes and reduce networking. An excess amount of competitor DNA, e.g. Cot-1 DNA, is commonly used to preferentially hybridise to (mask or block) repeat sequences making them less available for non-preferential probe/probe hybridisation and network formation. A disadvantage of Cot-1 DNA is that it masks only a proportion of repetitive sequences and there is evidence that it may actually stabilise the above mentioned networks. Another disadvantage of Cot-1 DNA is that it cannot be easily removed from final reaction products, in DNA based applications. The ability to remove such a blocker would be advantageous as it would promote the destabilisation of the above mentioned networks.

Common to the majority of hybridisation based approaches is the requirement for a solid surface: e.g. nylon membranes (Southern blotting); glass microarray surfaces (microarray based ROIs detection and hTE); and coated paramagnetic beads (used to recover probes in solution based hTE). However, DNA and RNA can interact with surfaces largely or completely irrespective of DNA sequence content, resulting in poor specificity when detecting ROIs, and the unwanted recovery of non-targeted molecules when enriching ROIs. Surfaces can be pre-treated with various blocking agents, such as bovine serum albumin (BSA) and polyvinylpyrrolidone (PVP), or even the DNA/RNA of an unrelated species. Blocking agents interact with the surfaces and thereby shield and prevent the surfaces from interacting with sample DNA/ RNA molecules.

For the above reasons, manufacturers prefer to produce specific, highly optimised, kits designed to recover pre-defined larger ROIs. So clearly there is still a need for improvements to hTE methodologies, to enable the highest possible enrichment power while reducing sensitivity to variations in ROI sequence properties, and improvements that lower the cost of hTE. This is particularly true for customisable hTE methods that target ROIs between a few tens of kb to a few Mb.

It is therefore an aim of embodiments of the present invention to overcome or mitigate at least one of the problems of the prior art.

It is also an aim of embodiments of the invention to provide methods of detecting and/or increasing enrichment of nucleic acid ROIs and to provide methods of cost effectively amplifying probes to detect ROIs.

Summary of the invention

According to a first aspect of the invention there is provided a method of nucleic acid sequence hybridisation comprising the steps of:

a) hybridising sample derived DNA that includes region of interest sequences with probe nucleic acid sequences; and

b) blocking or masking repetitive DNA sequences in the DNA by mixing the sample derived DNA that includes region of interest sequences with ribonucleic acid molecules comprising the same or substantially similar repetitive sequences as those of the sample derived nucleic acids before or during step a), wherein the mixing is carried out at between 50 °C and 80 °C.

The non-deoxy ribonucleic acid molecule may comprise RNA which is a transcription product from whole genomic DNA or from fractionated genomic DNA. The non-deoxyribonucleic acid molecule may be natural or synthetic.

There may be more than one non-deoxy ribonucleic acid molecule, or there may be one or more deoxyribonucleic acid molecule and at least one non-deoxyribonucleic acid molecule as blocking or masking agents. For example there may be a non-deoxyribonucleic acid and a DNA molecule as blocking or masking agents.

The RNA transcription product may be the transcription product from any prokaryote, eukaryote or archaea, for example mammalian DNA (including human DNA) or DNA of fish, reptile, bird amphibian, plant, fungal species. Suitable fish DNA includes salmon gDNA, or any combination thereof.

The or each RNA transcription product may be derived by transcription from whole genomic human DNA, human Cot-1 DNA, or salmon genomic DNA, or any combination thereof, for example.

In some embodiments, the RNA transcription product may comprise a mixture of RNA transcription products selected from mammalian DNA, fish DNA, bird DNA, reptile DNA, plant DNA and fungal DNA, such as a combination of mammalian and fish DNA, which may be RNA transcription products of whole genomic DNA. In some embodiments the combination comprises the RNA transcription products of human DNA and salmon DNA, especially of whole genomic human and salmon DNA.

It has surprisingly been found that utilising combinations of non-deoxy ribonucleic acids as blocking or masking agents may provide up to four or more times the enrichment power of DNA-based blocking or masking agents.

In another example, the blocking or masking may be effected by mixing a non-deoxyribonucleic acid molecule and a deoxyribonucleic acid molecule, with at least one sample nucleic acid, such as a mixture of an RNA transcription product of a DNA molecule and a Cot-1 DNA or salmon genomic DNA molecule, for example.

The RNA transcription product may later be eliminated from the reaction (e.g., from employed surfaces to which it has become bound, or from repetitive DNA fragments to which it has hybridised) by treatment with a removal agent, which may be an RNase (such as RNase A, RNase $I_f$ or RNase H, for example).

In an embodiment, the method comprises hybridisation of one or more sample derived nucleic acids comprising one or more regions of interest, the method comprising the step of hybridisation of each sample nucleic acid and/or region of interest with a plurality of non-overlapping nucleic acid probes.

A region of interest (hereinafter "ROI") is a contiguous genome nucleic acid sequence or non-contiguous set of genome nucleic acid sequences targeted for detection or recovery in an experiment. Examples of nucleic acids would include, DNA, hnRNA (heterogenous RNA), mRNA, tRNA or rRNA sequences.

"Sample derived nucleic acids" means one or more samples of nucleic acids from a biological sample or material.

A "probe" is a fragment or stretch of nucleic acid sequence, such as DNA or RNA, or a synthetic non-naturally occurring equivalent, which comprises the reverse complement sequence of either or both strands of a ROI. Probes that are recoverable are also known as "baits".

In some embodiments there are at least 50%, 60%, 70%, 80%, 90% or 95% non-overlapping probes used in the hybridisation method. There may be a plurality of over-lapping probes used in the hybridisation method, but they should preferably make up no more than 25%, 20%, 15%, or 10% of the total number of probes used in the methods of the invention, and in some embodiments no more than 10% or 5% of the total number of probes. The methods of the invention therefore utilise mutually largely non-overlapping probes for each ROI, in order to maximise hybridisation coverage of each ROI.

The method of the first aspect of the invention is particularly suited for use in hybridisation target enrichments (hTE) processes, and also in ROI detection methods.

The method may comprise hybridisation of a ROI that has been broken into a plurality of nucleic acid fragments. Each fragment may be as described herein below. These embodiments are particularly suited for hTE processes and therefore the method may comprise a process of hTE comprising hybridisation of one or more nucleic acid ROIs, with the method comprising the step of fragmenting the ROI nucleic acid sequences and hybridising the resulting fragments with a plurality of non-overlapping probes.

hTE methods require that a total nucleic acid sample is first fragmented into pools with fragment sizes of at least 500 bases, 700 bases, 900 bases, 1000 bases, 1200 bases, 1400 bases or 1500 bases. ROIs will be present within a subset of these fragments. It has been found that the method is particularly useful for recovering the ROI containing fraction from nucleic acid fragment pools with an average fragment size of between 900 bp and 1.2 kb, 1 kb and 1.5 kb, or between 1.1 kb and 1.4 kb, or between 1.2 kb and 1.3 kb.

In some embodiments the method of the first aspect of the invention comprises hybridisation of one or more nucleic acid fragments comprising at least a portion of one or more ROIs, wherein each fragment comprises at least 500 bases, 700 bases, 900 bases or 1000 bases. In some embodiments the method may comprise hybridisation of one or more nucleic acid fragments comprising at least a portion of one or more ROIs, wherein each fragment comprises no more than 2000 bases, 1800 bases, 1600 bases or 1500 bases.

In other embodiments the method of the first aspect of the invention may comprise a method of detecting a ROI. In such embodiments the ROI may comprise a relatively large number of nucleic acid bases, such as whole genes, for example.

The ROI may be greater than 50kb, 100 kb, 250 kb, 500 kb, 1 Mb or 2 Mb for example. In other embodiments of the invention the ROI may be at least 50Mb, 100 Mb, 150Mb or 200Mb.

In some embodiments the number of probes designed to hybridise per 1 kb of each nucleic acid ROI on average is at least 1 probe, at least 3 probes, at least 4 probes, or at least 5 probes. In some embodiments at least 3 probes, or at least 4 probes, or at least 5 probes are designed to hybridise per 1 kb of each ROI on average. In some embodiments there may be up to 20 probes designed to hybridise per 1 kb of each ROI on average.

In some embodiments the method may comprise, in addition to hybridisation of a plurality of non-overlapping probes to each ROI, hybridisation of portions of one or more probes to regions outside of and possibly flanking the ROI or ROI fragments. In some embodiments the method may comprise annealing portions of at least one probe to a region extending up to 100bp, 200bp or 300bp outside of and possibly flanking the ROI or ROI fragments. These embodiments are particularly suitable for methods of hTE in which it is important to ensure efficient recovery of all sub-regions of an ROI.

In the first aspect of the invention, the hybridisation methods described herein have been found to provide a number of advantages over known hybridisation enrichment or detection methods. With respect to hTE the method enables accurate recovery of relatively long (800-1500 bp) target nucleic acid fragments that contain ROIs using a plurality of non-overlapping probes, compared to current techniques which utilise shorter target nucleic acid fragments (200-500bp) and a plurality of frequently overlapping probes. The use of longer target nucleic acid fragments in the method: leads to more efficient recovery of ROI bases situated near to junctions with non-ROI bases; increases the uniformity of recovery throughout ROIs; promotes better recovery of "difficult" regions such as regions with secondary structure or particularly high or low proportions of C+G base content; and maximises the number of base pairs formed between probes and ROI nucleic acids, which thereby increases resistance to stringent washing and so improves the specificity of product recovery. The use of a plurality of non-overlapping probes in the method counters problematic steric hindrance and competition at regions where probes overlap.

In some embodiments the probes hybridise with at least 50%, 60%, 70%, 80% or 90% of the length of a given ROI. In some embodiments, the probes hybridise with at least 95%, 96%, 97%, 98% or 99% of the length of a given ROI.

In some embodiments at least one probe or bait is annealed within 5, 10, 15, 25, 50 or 100 bases from an end of each ROI or each ROI fragment. In some embodiments at least one probe is annealed within 5, 10, 15, 25, 50 or 100 bases from both ends of each ROI or each ROI fragment.

In some embodiments at least 5%, 10%, 15% 20%, 25%, 30%, 40% or 50% of the probes are non-overlapping on the ROI or each ROI fragment. In some embodiments at least 75%, 80%, 85%, 90% or 95% of the probes are non-overlapping on the ROI or each fragment ROI fragment. In one embodiment 100% of the probes are non-overlapping.

In some embodiments the method comprises immobilising the probes onto a surface to provide a microarray of immobilised probes. The method may comprise hybridising sample nucleic acids including the ROIs to a plurality of the immobilised probes, followed by washing, to preferentially denature and remove non-ROI derived and non-annealed nucleic acids.

In other embodiments the method may comprise in-solution hybridisation, wherein the probes and ROIs are first hybridised in-solution. The probes may be labelled with biotin or any other suitable tag or label and recovered using Streptavidin coated, or otherwise suitably coated, paramagnetic or other beads or other suitable coated solid surface, to facilitate the recovery of these surfaces and the nucleic acids attached to them. The method may then comprise the application of stringent wash conditions to preferentially remove non-hybridised or non-specific hybridised nucleic acid.

In some embodiments, the first aspect of the invention comprises a method of hTE of a ROI. In other embodiments the first aspect of the invention comprises a method of detection of a ROI.

In some embodiments, at least one nucleic acid probe may be labelled with a plurality of the same or different labels per probe molecule.

In some embodiments at least one probe nucleic acid comprises at least 6, 8, 10, 12, 14 or 15 labels per molecule.

In some embodiments at least one nucleic acid probe comprises a label within 10, 5, 3, 2, 1 or 0 bases from an end of the nucleic acid probe. This could be an end of a probe that comprises additional bases not designed to hybridise with any ROI bases. Such non-targeting ends of the nucleic acid probe(s), if included, may comprise the 5' end or the 3' end or both ends of the molecule, and the label may be placed within 10, 5, 3, 2, 1or 0 bases of such an end. With respect to recoverable probes the label is typically an entity that facilitates physical recovery of the label and the nucleic acids adjoined to it. The 3' end of the probe may comprise a dideoxynucleotide so as to prevent polymerase based extension, and thereby enable polymerase chain reactions to be used to amplify and hence recover target sequences that have been captured.

Each label may independently comprise a fluorescent marker, a luminescent marker, a recoverable marker, a radioactive marker, or the like.

Each label may independently comprise biotin.

The method may comprise using a plurality of the above described probes and in some embodiments all of the probes used are as described above. In some embodiments of the invention the method may comprise using a plurality of non-overlapping probes as described above.

The probe or probes as described above ensure that their use in hybridisation events creates target-probe duplex structures in which multiple copies of the label are present, which facilitates improved ease and strength of detection or recovery.

Included non-targeting ends may comprise at least 2, 3, 4, 5, 6, 7, 8, 9 or at least 10 labels. In some embodiments the non-targeting ends may comprise more than 10 labels.

The method may further comprise the use of a non-deoxy ribonucleic acid molecule to block or mask a surface.

Surface blocking or masking agents or repetitive DNA blocking or masking agents may be manufactured by the following method:

a) fragmentation of DNA;

b) transcription of the DNA fragments to provide a mixture of RNA transcription products;

c) removal of residual DNA with DNase; and

d) removal of DNase with a proteinase.

Step b) may typically include ligating the target fragments to DNA sequences that encode one or more RNA polymerase promoters (such as T7), amplification procedures, and incubation in the presence of RNA polymerases to transcribe the DNA.

The DNase may be DNase I. The proteinase may be proteinase K.

There may be a step e) of purifying the RNA transcription product and protecting the product by addition of a reversible RNase inhibitor.

In some embodiments the method may comprise adding two or more non-deoxy ribonucleic acid molecules, such as a combination of RNA transcription products. In preferred embodiments the method comprises adding the RNA transcription product of mammalian DNA and fish DNA, such as a combination of the transcription products of human DNA and salmon DNA, preferably of whole genomic DNA.

During hybridisation of target DNA sequences to probes, repetitive sequences within the sample DNA and/or probes may give rise to unwanted hybridisation events involving ROI and/or non-ROI related sequences. Such hybridisation between repetitive sequences can create networks of DNA fragments which can lead to unintended detection or recovery of non-ROI based sequences. To counter this tendency, repeat sequence containing blocking reagents such as Cot-1 DNA may be added during hybridisation to bias network formation towards interactions between sample derived DNA fragments and blocker molecules rather than only between sample derived DNA fragments. Multiple target derived DNA fragments are therefore less likely to become joined together in any one network, and so this minimises the recovery or detection of non-ROI sequences. Furthermore, including repeat sequence blockers comprised of non-deoxy ribonucleic acids (such as a genomic DNA derived RNA transcription product) in the hybridisation process, and following this by treating with RNase, serves to break up repetitive element networks, so that subsequent washing is able to remove much of the destroyed network and hence reduce the level of detection or recovery of non-ROI based sequences.

The method may comprise hybridisation of a nucleic acid ROI with a plurality of probes and addition of a non-deoxy ribonucleic acid molecule, such as a RNA transcription product of genomic DNA, during the hybridisation reaction. The probes may be as described above and may comprise one or more probes having multiple labels as described hereinabove. It has been found that hybridisation using such probes as described above typically produces an EP of at least 250 and highly uniform rates of non-repetitive sequence recovery across an ROI, whilst being cost effective compared to related competing technologies. When such methods of hybridisation using the probes described above are also combined with the method of the seventh aspect of the invention, the EP increases to >2000 which is believed to match or exceed the capabilities of alternative contemporary market-leading hTE methods.

In some embodiments the method may further comprise blocking a solid surface comprising the steps of

a) optionally washing the solid surface with hybridisation buffer

b) incubating the solid surface with hybridisation buffer containing a blocking reagent

c) adding at least one probe as described hereinabove.

The blocking reagent in step b) may be a nucleic acid or synthetic non-natural equivalent and may be a non-deoxy ribonucleic acid as described hereinabove. The blocking reagent may be a transcription product from any prokaryote, eukaryote or archaea, for example mammalian DNA (including human DNA) or DNA of fish, reptile, bird amphibian, plant, fungal species. Suitable fish DNA includes salmon gDNA. The blocking aspect may comprise a surface masking blocking agent manufactured according to the method described hereinabove.

Common to the majority of hybridisation based methods is the involvement of a solid surface, such as a nylon membrane (e.g., as in Southern blotting); glass surfaces (e.g., as in microarray-based ROI detection and hTE); or coated paramagnetic beads (e.g., as used to recover probes in solution-based hTE). DNA and RNA can form interactions with surfaces, resulting in non-specific signals when detecting ROIs and the recovery of non-ROI sequences when enriching ROIs. Surfaces can be pre-treated with blocking agents, such as bovine serum albumin (BSA) and polyvinylpyrrolidone (PVP), or even the DNA/RNA of an unrelated species. Blocking agents interact with the surfaces and thereby shield the surface from interaction with and binding to the sample DNA/RNA, hence significantly reducing the detection or recovery of unintended DNA sequences.

The method may further comprise amplification of short, mixed nucleic acid sequences, comprising the steps of:

a) providing between around 1fg (femtogram) to around 500pg (picogram), though preferably between around 1pg and around 250pg, of a complex pool of single-stranded nucleic acid fragments having common sequences at their 5' ends and having common sequences at their 3' ends.

b) amplifying the nucleic acid fragments, preferably by polymerase chain reaction with a suitable primer or pair of primers.

In some embodiments the nucleic acid fragments in step a) have a length of $\leq$ 1.5kb, $\leq$ 1kb, less than 500 bases, less than 400 bases, less than 300 bases, less than 250 bases, or less than 200 bases. In some embodiments the nucleic acids in step a) have a length of between 60 and 250 bases, or between 80 and 200 bases, or between 100 and 200 bases.

Step b) should be undertaken such that there is no significant change to the diversity of the complex pool.

The nucleic acid fragments in step a) may have a plurality of common sequences at their 5' ends and/or their 3' ends.

It has been found that one can effectively and with high fidelity amplify these nucleic acid sequences, such as the probes described hereinabove, by starting from such tiny amounts of this type of starting material, in the femtogram to picogram range (which is far less than is generally used in such amplifications). When higher amounts of such short nucleic acid fragments are used in standard PCRs the molecules tend to interact with each other such that 3' ends become non-specifically hybridised and then extended by copying whatever other sequence to which they had spuriously hybridised. As the PCR cycles progress, progressively more and longer spurious products are generated. However, if the starting concentration of these PCR targets is low, they will instead be preferentially primed as intended by primers matched to their common ends, and the desired products thereby amplified. This noted problem with such PCRs is vastly exaggerated when the starting mixture of short fragment targets is derived by synthesis on microarrays. Such array-derived DNA pools often contain a very high proportion of truncated molecules (~30% to ~99%) (LeProust et al., 2010), which therefore cannot be primed and amplified as intended, but can become involved in cross-priming with other target fragments.

In some embodiments step a) comprises providing at least 10fg, 100fg or 500fg of nucleic acid fragments. In some embodiments step a) comprises providing no more than 450pg, 400pg, 350pg or 300pg or nucleic acid fragments.

The probes and blocking or masking reagents described hereinabove may also be used in other applications such as fluorescence in situ hybridisation (FISH), for example.

Detailed Description of the Invention

Embodiments of the various aspects of the invention will now be described by way of example only, with reference to the accompanying drawings of which:

Figure 1 shows the structure of single-stranded array synthesised DNA produced in Example 1. The top panel shows full-length short (<200 bp) single-stranded DNA molecules (oligonucleotides), and the bottom panel shows the structure of a truncated oligonucleotide (note, the length of truncated oligonucleotides are variable) lacking the 5' primer annealing site.

Figure 2 is an Agarose gel image showing PCRs seeded with serially diluted complex pools of oligonucleotides (complex pools). The PCRs were seeded with 100 pg, 10 pg and 1 pg of the complete range of high to low quality complex pools (100% to 0.1%). The DNA marker ladder is a 50 bp ladder (NEB).

Figure 3: Left shows an agarose gel image showing PCRs seeded with serially diluted complex pools (as seen in Figure 2). Right shows PCRs seeded with either 100% full length complex pool (black) or equivalent effective masses of a 10% or 1% complex pool. The DNA marker ladder is a 50 bp ladder (NEB) on the left, and a 100 bp marker ladder (NEB) on the right.

Figure 4 illustrates a representation of a hybridised DNA ROI with multiple probes usable in the invention;

Figure 5 illustrates a representation of an embodiment of the probe usable in the invention;

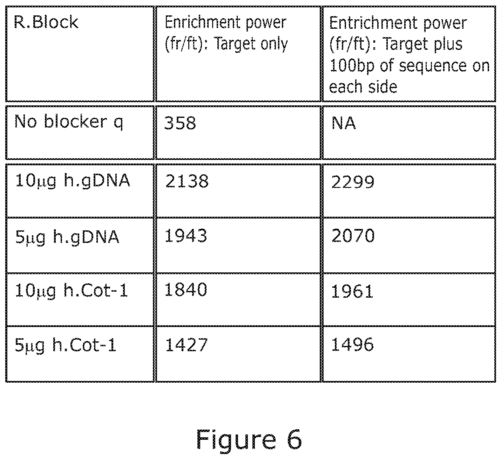

Figure 6 is a table showing the enrichment powers achieved by in-solution hTE using various non-deoxy ribonucleic acid molecules in the form of RNA transcription products of various DNA fragments (hereinafter "R.Block") provided by the methods of the sixth and seventh aspect of the invention. Enrichment power (EP) is the fraction of resulting on-target NGS reads over off target reads (fr) divided by the fraction of the genome that has been targeted (ft). Column 1 shows the source of the DNA fragments used to produce each R.Block. Column 2 shows enrichment powers for reads overlapping the target. Column 3 shows the enrichment power for reads overlapping the target plus 100 bp of sequence on each side;

Figure 7 illustrates a target DNA sequence hybridised with probes in which repetitive elements within the DNA sequence have formed a repetitive element network; and

Figure 8 is a graph showing the enrichment power (EP) conferred when using various repetitive sequence blocking agents and combinations, as described in Example 13.

**Example 1 - In-solution complex pool PCR**

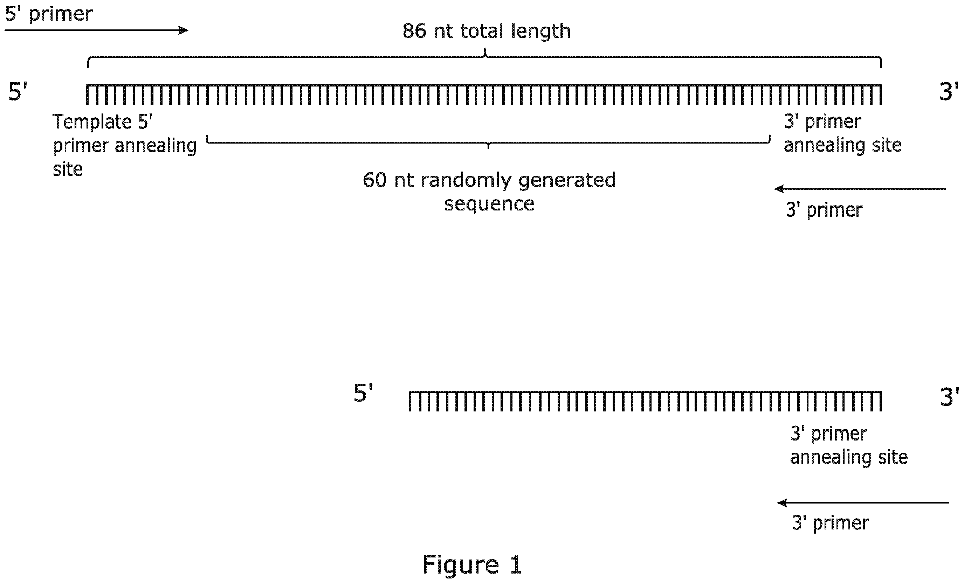

An optimised method for the amplification of complex pools containing array-synthesised short (<200 bp) single-stranded DNA molecules was developed. A 'model' pool (produced by conventional long oligonucleotide synthesis) was used to evaluate various reaction parameters. The model pool as shown in Figure 1 was designed to accurately represent complex pools of array-synthesised single-stranded DNA molecules, and it consisted of: a 9 nt, 13 nt or 20 nt template 5' primer annealing site; a run of 60 (or more) randomly incorporated nucleotides (representative of the hundreds of thousands of unique sequences available during array synthesis); and a 9 nt, 13 nt or 20 nt 3' primer annealing site. Terminal primer annealing sites of 13 nt were used to maximise the "unique sequence" capacity of the single-stranded DNA molecules. The complex pool was purified by Polyacrylamide Gel Electrophoresis (PAGE) and High Pressure Liquid Chromatography (HPLC) by Biomers-net GmbH (Germany) (Biomers) to ensure that it had a quality score of ~100% based on the percentage of full length molecules compared to truncated molecules. The complex pools were then mixed with a truncated version of the same pool which lacked the 5' template primer site to produce pools containing 100%, 50%, 10%, 1% and 0.1% of the full length molecules respectively.

**Selecting a suitable system for complex Pool PCR**

All PCRs were prepared on ice. 30-50 $\mu$l PCRs contained 1x of the supplied PCR Buffer, 0.15 pmols/ul ProAmpF04E, 0.15 pmols/ul ProAmpR01D, 0.2 mM dNTPs, 0.025 $\mu$ U/$\mu$l of the required DNA Polymerase, and the required mass of mixtures of full length and truncated single-stranded DNA molecules. Reactions were sealed with a heat sealable PCR film or PCR strip-caps (Thermo fisher Scientific, Loughborough, Leics, UK).

Optimal thermal cycling conditions were determined to entail the following: 98°C for 30 sec, 5x (98°C for 30 sec, 65°C for 10 sec), 25x (unless stated elsewhere) (98°C for 10 sec, 70°C for 10 sec) 72°C for 1 minute then held at 15°C. Following cycling, 10 $\mu$l of the PCRs were subject to electrophoresis alongside 1 $\mu$g 50 bp ladder (NEB, Hitchin, Herts, UK) on a 2.5% LE agarose gel stained with 0.2 $\mu$g/ml EtBr. Completed PCRs were stored at -20°C. The 5°C reduction in annealing temperature for the first 5 PCR cycles allowed the primers to initially anneal to the primer annealing sites <20 nt in length. Once these had been extended by polymerase extension, the annealing temperature could be raised to 70°C.

A wide range of DNA polymerases were evaluated including: Amplitaq Gold (Applied Biosciences), Pfu Ultra high fidelity DNA polymerase (Agilent), Phusion high fidelity DNA polymerase (NEB), iProof high fidelity DNA polymerase (BioRad) and Velocity (Bioline). These investigations showed that iProof High-Fidelity DNA polymerase (BioRad) worked particularly well for complex pool amplification, along with Phusion and Velocity.

These methods produced a robust and greatly improved method for complex pool amplification. But beyond the PCR conditions, other factors (complex pool quality and complex pool quantity) were also found to be of great importance, as described below.

**Example 2: Emulsion-Based complex Pool *PCR***

Emulsion PCR (EMPCR) has been proposed as a means to improve troublesome PCRs, especially if they involve complex template DNA mixtures. EMPCR entails creating, in one tube, millions of femtolitre sized droplets of oil-coated water (including PCR buffer, primers etc), such that each of these volumes acts as a separate reaction vessel within which PCR amplification can occur starting from a few template molecules. Since this arrangement reduces the chances of cross-priming and other undesirable interactions between different templates and their products, there is theoretically a limited risk of generating many different false products. Also, should cross-priming occur, the encapsulation limits the resources available to the un-desirable product thus preventing over amplification. This does not, however eliminate the possibility of false internal priming within synthesized strands (by primers or products strands), or concatamerisation between single-stranded amplification products, within each sub-reaction. Nevertheless, EMPCR has been adopted by many researchers to try to improve the effectiveness of challenging complex pool amplifications in order to enhance product quality.

To test the actual effectiveness of EMPCR for complex pool amplification, 30μl PCRs were seeded with lOng of the model complex pools, and standard HF buffer (BioRad) was replaced with a detergent free formulation of the same buffer (BioRad) to prevent dispersal of the emulsion. Emulsification was performed by overlaying the reactions with 170 μl of a pre-prepared and chilled mixture containing 73% Tegosoft DEC (Evonik, Essen, Germany) 3% Abil WE09 (Evonik, Essen, Germany) and 20% Light mineral Oil (Sigma Aldrich, Gillingham, Dorset, UK), followed by transfer into a 4°C constant temperature room and shaking at maximum speed on a vortex device for 10 min. EMPCRs were then performed for 20 to 30 thermal cycles.

The emulsions were broken by addition of 500 μl Butanol (Thermo Fisher Scientific) and the samples briefly vortexed. Then, 150 μl of buffer PB (Qiagen, Crawley, West Sussex, UK) was added and mixed into each sample by brief vortexing. Products were recovered from the whole sample by purification upon Qiagen MinElute PCR columns according to the manufacturer's protocols. Purified reaction products were eluted in 30 μl buffer EB (Qiagen).

Agarose gel electrophoretic analysis of the products revealed that the amplified DNA fragments were all of the desired size range (a single gel band), but that each 10 μl PCR volume was able to generate only a few ng of material, no matter whether the reactions were seeded with a high quality (100% equivalent to lOng of amplifiable full-length molecules) or a low quality (0.1% equivalent to 100pg of amplifiable full-length molecules) complex pool template.

Another downside with EMPCR relates to the unavoidable cost, time and complexity of the process of emulsion breaking and subsequent product purification. Solution phase PCRs can be de-salted and purified by running through a chromatography column (e.g., Microbiospin, BioRad) or micro filter column (e.g., Amicon Ultra, Millipore). But to purify EMPCRs, special columns are required to remove the emulsion oils. Such columns are more likely to allow passage of contaminants such as ethanol and chaotropic salts into the eluted product.

These results show that EMPCR can amplify lOng of a complex pool without generating excessive amounts of spurious product, however the poor PCR dynamics within the emulsion cause the approach to generate far too little material for the needs of most downstream applications.

**Example 3 - Improved complex pool PCR usable in the invention**

Spurious products in complex pool PCR may be caused by 'over-cycling'; especially since the problem worsens as the total number of thermal cycles increases. The concentration of genuine product will rise so high in the later cycles that DNA strands can; a) start to cross-prime onto each other, generating false longer products, and b) become available for internal priming by the common primers, generating false shorter products. However this hypothesis fails to explain why the same type of events would not also occur for many of the amplified target sequences within their individual droplets in EMPCR.

The problem may be triggered by events that occur towards the start rather than at the end of the PCR, especially in PCRs with an excessive starting concentration of complex single-stranded DNA molecules. These events then create a low background of various artefacts some of which could amplify as efficiently as genuine products, such that they come to dominate the genuine products as more and more reaction cycles are performed. The nature of these initial 'trigger' events would also have to be such that they cannot occur (or are very much minimized) in the EMPCR context, wherein the target molecules are mostly isolated from one another into small clusters within the oil droplets.

A PCR seeded with lOng of human genomic DNA will have within it few free 3' ends and only $\sim 6 \times 10^3$ amplifiable target strands (lOng / 3pg (Mass of a single haploid genome) x 2 (to convert to single-stranded molecules)). In contrast, a PCR seeded with lOng of an complex pool of short single-stranded DNA molecules (which is perhaps up to 10% of the original pool that will have been supplied/purchased) will contain $\sim 2 \times 10^{11}$ amplifiable molecules, with an equally large number of free 3' ends. This $>> 10^7$ fold relative excess is enormous, and it means the starting situation of an complex pool PCR is analogous to the situation that will exist in a regular genomic DNA target PCR at the end of the whole reaction ($\sim 25$ cycles). These almost 1 trillion targets therefore represent a mass of diverse sequence primers which can diffuse quickly and use their free 3' ends to prime on other molecules, and since it is also composed of a myriad of different sequences there will be great potential for internal cross-priming of one sequence onto another. It is therefore believed that the cross-priming and mis-priming events towards the end of an 'over-loaded' complex pool PCR probably start happening excessively in the very first few cycles of such PCRs. This creates various artefacts that then further amplify and can eventually outnumber the desired products as the amplification of the desired products plateaus during the later stages of the PCR. This problem does not exist in EMPCR, since the original templates are physically separated from one another from the start, and the resources contained within each reaction droplet are very limited Furthermore, the overall negative impact of this undesirable mis-priming and inter-molecule priming is likely to be proportional to the fraction of target molecules that are full length (not truncated at their 5' end), as only this class of original template can be internally primed and copied to generate an artefact with a common priming site at its newly synthesised (3') end.

In order to overcome these problems with complex pool PCR a method was performed according to the tenth aspect of the invention in which a significantly reduced amount of template pool was used.

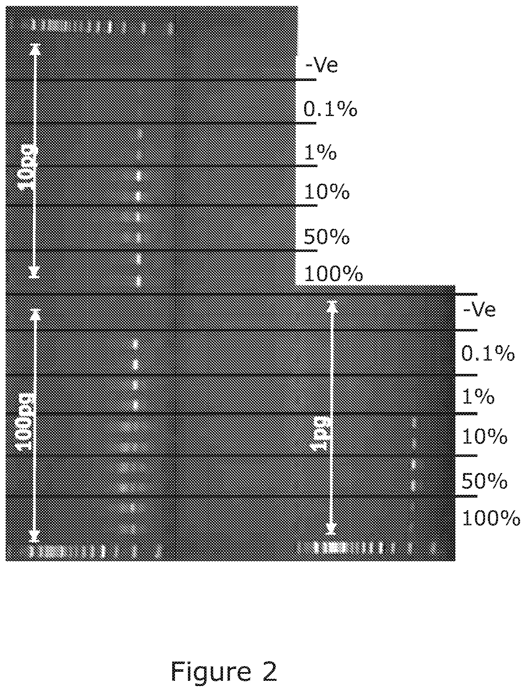

Duplicate PCRs were performed in $30\mu$ $\mu$l volumes seeded with 1$\mu$l of 10x serial dilutions of each of the different quality model pools, using 30 thermal cycles. The input pools contained lOng, 1ng, 100pg, 10pg and 1pg of, single-stranded DNA molecules. Example results from such experiments using the optimum enzyme and reaction conditions, detailed above in Example 1, are shown in Figure 2. Agarose gel images from reactions that employed 10 ng or 1 ng of input material are not shown, as they contained nothing but excessive amounts of spurious amplification products. However, the results were greatly improved for reactions that used lower amounts of input complex pool.

As can be seen in Figure 2, for reactions seeded with 1 pg of total template, the 10%-100% quality models amplified the desired fragment mixture very cleanly. Thus 0.1-1 pg of full length target was sufficient, and 0.9 pg of truncated target did not compromise these reactions.

For reactions seeded with 10pg of total template, the 1%-100% quality models amplified the desired fragment mixture very cleanly. Thus 0.1-10 pg of full length target was sufficient, and 9.9 pg of truncated target did not compromise these reactions.

For reactions seeded with 100 pg of total template, the 0.1%-1% quality models amplified the desired fragment mixture very cleanly. Thus 0.1-1 pg of full length target was sufficient, and 99.9 pg of truncated target did not compromise these reactions. However, the reaction with 10 pg of full length target was compromised (overtaken by artefacts) by the presence of 90 pg of truncated target. The reactions with 50 pg and 100 pg of full length target also generated a lot of artefacts.

These results suggest that the main factor that determines whether artefacts are formed in an complex pool PCR is the absolute amount of full-length target present at the start of the reaction. To ensure good quality amplifications, this quantity should be of the order of 1 pg, though it is quite robust to an order of magnitude difference up or down. The amount of 5' truncated target has a far smaller influence of the reaction fidelity, even up to the 10-100 pg range - though if more than this is present in the starting reaction then more artefacts will be produced, and a mild excess of truncated template seems to cooperate with a mild excess of full length template in generating undesirable products.

### *Optimisation to reduce PCR cycle number*

Performing fewer PCR cycles reduces the likelihood of errors within the amplified sequences. Q5 polymerase (NEB) has an error rate >100 fold lower than Taq DNA polymerase which relies on efficient 3' to 5' exonuclease activity. However, the efficient 3' to 5' exonuclease activity also degrades primers during PCR (Pers. Comm, NEB technical support). Using a single Phosphorothioate bond at the 3' end of PCR primers would prevent 3' to 5' exonuclease activity but would also block desirable exonuclease activity e.g. $\lambda$ exonuclease.

A series of PCRs with differing primer concentrations in which complex pools with quality scores of 10 and 1% were carried out using the following conditions: 50 $\mu$l PCRs contained 1x of the supplied PCR Buffer, 1.5 pmols/$\mu$l ProAmpF04E, 1.5 pmols/$\mu$l ProAmpR01, 0.2 mM dNTPs, 0.025 $\mu$ U/ $\mu$l of Q5 DNA Polymerase, and the required mass of template pool). Reactions were sealed with a heat sealable PCR film or PCR strip-caps (Thermo fisher Scientific, Loughborough, Leics, UK). The reactions were cycled as follows: 98°C for 30 sec, 5x (98°C for 30 sec, 65°C for 10 sec), 20x (98°C for 10 sec, 70°C for 10 sec) 72°C for 1 minute then held at 15°C. Additionally, the PCRs were performed with and without supplementing with 0.01 $\mu$ U/$\mu$l of Thermostable Pyrophosphatase (NEB)

Increasing the primer concentration in the PCRs and supplementing with Thermostable Pyrophosphatase increased the yield that could be generated per 50$\mu$l PCR from ~0.2 $\mu$g to >0.5 $\mu$g using 5 to 6 fewer PCR cycles and 10 fold less template.

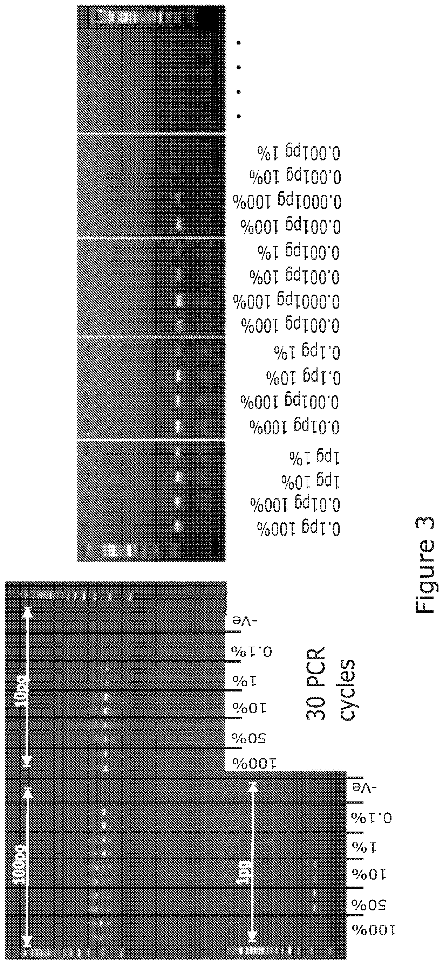

Further optimisations showed that equivalently high yields could be achieved by seeding PCRs with 10 pg to 20 pg of the complex pool and performing 15 to 17 PCR cycles (Figure 3).

*Comparison with EMPCR*

The above in-solution complex pool PCR technique of the invention is shown to be more efficient than EMPCR.

Considering a complex pool with a quality score of ~70% and a yield of ~300 ng:

1. Optimised EMPCRs would be seeded with a few ng of complex pool and yield ~200 ng per PCR. The maximum expected yield from PCR of the whole complex pool would be ~120 μg.
2. The PCRs produced using the inventive method of Example 3 would be seeded with 1 pg to 20 pg of the template complex pool and yield >500 ng per PCR. The maximum expected yield from PCR of the whole complex pool would be >1 mg

The inventive technique is also faster to set up, as EMPCR requires a long emulsification step prior to thermal cycling, and a long demulsification step following thermal cycling.

The inventive technique allows easier purification of PCR products, as it is compatible with a wide range of purification platforms e.g. Silica membrane columns (Qiagen), Silica coated beads (Qiagen), AmPure XP beads (Beckman), and Polyacrylamide gel buffer exchange (BioRad) etc. The emulsifying oils used for EMPCR limit compatibility with some of these purification platforms.

EMPCR is also intolerant of soap containing buffers. iProof polymerase has a soap free buffer available, but many other polymerases such as Q5 polymerase are optimised for use in soap containing buffers. The inventive technique is compatible with a range of buffers. The inventive technique may be adapted to amplify ng masses of complex pools.Finally, since EMPCR compartmentalises the reaction, it is possible that the separate compartments might consume their resources at different rates and may result in an un-even product.

## Example 5 - Generating multi-biotinylated double-stranded PCR amplicons usable in the invention

Complex pool pool PCRs were performed using standard optimised conditions but with substitution of dCTP for differing ratios of 17 Biotin-16-Aminoallyl-2'-dCTP and dCTP (Trilink). Biotin-16-Aminoallyl-2'-dCTP has a flexible linker arm making it more efficient for use in PCR than other biotinylated nucleotides. It was found that a ratio of 0.65 17 Biotin-16-Aminoallyl-2'-dCTP gave an optimal balance between yield and biotin incorporation.

## Example 6 - Producing a multi-biotinylated probe library from a template complex pool

PCR amplified complex pools are double-stranded. To generate multi-biotinylated probes for a hybridisation based target capture, the multi-biotinylated double-stranded pool was transformed into a single-stranded pool. To achieve this, the 3' primer site was removed with the *Bts* I restriction enzyme (NEB) and the unwanted strand removed with λ-exonuclease (NEB).

To allow direct PCR recovery of captured DNA fragments following targeted enrichment, it was necessary to protect the 3' end of the probes from primer extension by DNA polymerases. Terminal Transferase (NEB) was used to add di-deoxy ATP (ddATP, Trilink) to the 3' end of the probe strands prior to the removal of the un-desired strand by λ-exonuclease.

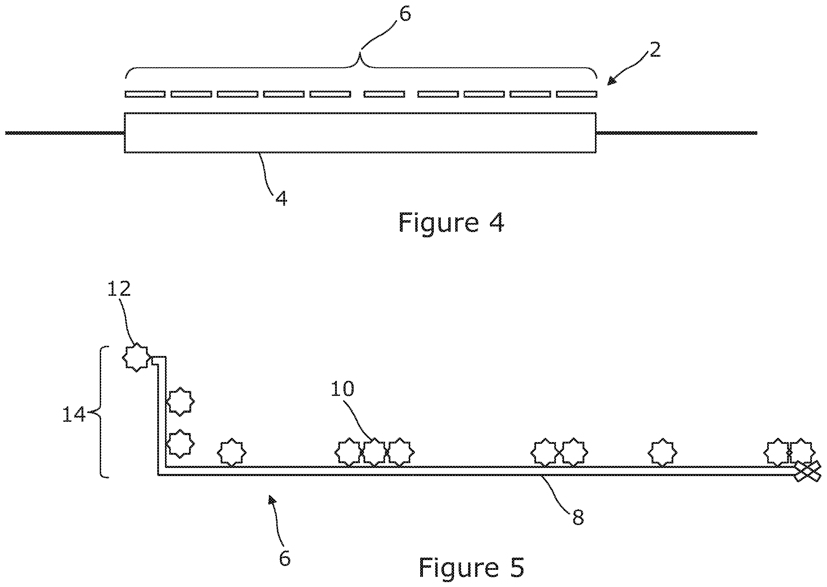

The output from processing is a pool comprising single-stranded multi-biotinylated probe with a non-target end as shown in Figure 5.

The multi-biotinylated DNA probe of the third aspect of the invention produced for example by the method described above has several potential advantages over existing DNA and RNA probes:

1. DNA probes are less vulnerable to environmental nucleases than RNA probes.
2. DNA probes are more compatible with many blocking or masking agents including those described in relation to the fourth to eighth aspect of the invention.
3. Multi-biotinylation presents numerous targets for Streptavidin binding making capture more efficient.
4. The non-target end region ensures that at least one biotin is distal to the targeting region.

The method of producing the multi-biotinylated probe library was as follows:

### a. PCR amplification of a template probe library

The template probe was diluted in lOmM Tris HCl (pH 8.5). A PCR master mix sufficient for ~100 PCRs was prepared containinglx Q5 high fidelity PCR buffer (NEB), 1.5 to 3 pmol/ul of 5' biotinylated ProAmp-F primer, 1.5 to 3 pmol/ul 5' phosphorylated ProAmp-F primer, 3 μM dGTP, 3 μM dATP, 3μM dTTP, 105 μM dCTP, 195 μM Biotin-16-

AA-CTP (Trilink), 0.02 U/µl Thermostable inorganic Pyrophosphatase (NEB), 0.05 U/µl Q5 hot start high fidelity DNA polymerase (NEB). The master-mix was vortexed.

Several 49 µl DNA free controls were aliquoted to which 1µl of water was added. The template pool was added to the remaining master-mix to a concentration of 0.02 to 0.4 pg/µl. Following vortexing, the master-mix was aliquoted into 50 µl reactions and the PCR tubes sealed. The reactions were cycled as follows: 98°C for 30 sec, 5x (98°C for 30 sec, 65°C for 10 sec), 10 to 20x (98°C for 10 sec, 70°C for 10 sec) 72°C for 1 minute then hold at 15°C. Following PCR, samples are stored at -20°C.

### b. Purification and concentration of PCRs

Several PCRs were pooled and vortexed. 200 to 500µl aliquots of the pooled PCRs were purified using MinElute columns (Qiagen) using the standard operating procedure, ensuring that the binding capacity of the column was not exceeded, with the following exceptions: All centrifugations were performed at 16000 RCF. Elution buffer (EB - 10mM Tris HCl pH 8.5) was heated to 70°C. 10µl of heated EB was added directly to each column followed by a 5 min incubation at 70°C. The eluate was recovered by centrifugation. A further 10 µl of pre-heated EB was added to each column, incubated for 1 min at 70°C and the eluate recovered by centrifugation. Following purification, all eluates were pooled and vortexed.

### c. Quantification and quality assessment of PCR

A DNA 1000 chip for the bioanalyser 2100 (Agilent) was used to assess the quality of the amplification. A single broad peak (broad due to the random incorporation of Biotin-16-AA-CTP) was identified with the crest of the peak at ~200 bp. The increased peak size was caused by retardation of the PCR fragments due to incorporation of Biotin-16-AA-CTP.

Following bioanalyser 2100 analysis, the concentration of the amplified complex pool was determined using a NanoDrop spectrophotometer (Thermo).

### d. Resolution of PCR into a single-stranded probe library

The total Mass of the amplified complex pool was determined. A reaction was prepared on ice such that every 20 µl contained 2 µg amplified complex pool, 1x Terminal Transferase buffer (NEB), 1x $CoCl_2$ (NEB), 0.125 U/µl *Bts*I, 0.2 µg/µl BSA (NEB) and 500µM ddATP (Trilink). The reaction was mixed by vortexing and incubated for 30 min at 55°C. The reaction was incubated on ice for 5 min.

3 µl of a mixture containing 2.5 µl of Terminal Transferase at 20,000 U/ml (NEB) in 1x Terminal Transferase buffer was added per 20 µl of the reaction. The reaction was vortexed to mix and incubated for 60 min at 37°C. The reaction was incubated on ice for 5 min.

3 µl of a mixture containing 2.5 µl of λ exonuclease at 5000 U/ml (NEB) in 1x Terminal Transferase buffer was added per 20 µl of the initial reaction volume. The reaction was vortexed to mix and incubated for 20 min at 37°C and 20 min at 80°C.

### e. Purification of the probe library

Sufficient MicroBioSpin p6 columns (BioRad) were warmed to room temperature such that 75 µl of un-purified probe library could be passed through each column. The probe library was purified according to the manufacturer's standard operating procedure. Following purification, the eluates were pooled and gently vortexed.

### e. Quantification and QC of the purified probe library

The purified probe library was analysed using an RNA 6000 nano chip for the Bioanalyser 1100 (Agilent) and quantified using a NanoDrop spectrophotometer (Thermo) An ideal probe library should have a concentration of ≥50 ng/µl and an OD 260:280 of 1.7 - 2.0.

### Preparation of human gDNA fragment libraries

### gDNA fragmentation

This method describes fragmentation using a Bioruptor sonicator. Note: Other DNA fragmentation options may be implemented, for example the Covaris system (Covaris), nebulisation (Roche), or by NEBNext dsDNA Fragmentase (NEB).

The gDNA was diluted in 10 mM Tris HCl (pH 8.5) to a concentration of 20 ng/μl. 110 μl of the diluted DNA was aliquoted into separate 1.5 ml sonication tubes (Diagenode), vortexed and centrifuged briefly prior to incubation on ice until the Bioruptor (Diagenode) was prepared.

To prepare the Bioruptor, the shearing bath was chilled for 30 min with water containing an ~0.5 cm layer of crushed ice. Following preparation, the aliquots of gDNA were placed into the Bioruptor's sample cradle and device assembled according to the manufacturers guidelines.

The samples were sonicated as follows:

| | |
|---|---|
| Power setting | Low |
| Sonication cycle | 15 sec on followed by 90 sec off |
| Number of cycles | 5-25 (dependant on the required level of sonication) |

Following sonication the fragmented DNAs were pooled and stored at -20°C.

**Small fragment removal purification of the DNA fragments**

Aliquots of the pooled sheared gDNA were purified using 1.2x to 1.8x AmpureXP beads (Beckman Coulter), dependant on the required fragment size, according to the manufacturers standard operating procedure. Finally the DNA was eluted in 10 mM Tris HCl (pH 8.5) with incubation at 65°C for 5 min prior to removal of the magnetic beads. Purified sheared gDNAs were quantified using a NanoDrop spectrophotometer and the fragment size determined using a DNA 7500 chip for the bioanalyser 2100 (Agilent). Purified sheared gDNA was stored at -20°C.

**Fragment polishing, dA tailing and linker ligation to produce a raw template library**

25 μl reactions were prepared on ice containing 500ng to 1000ng of fragmented gDNA, 1x Thermopol buffer (NEB), 2% PEG 4000 (Fermentas) 1.0 mM ATP (Thermo), 0.4 mM dNTPs (Promega) 0.4 U/μl T4 polynucleotide kinase (Fermentas), 0.1 U/μl T4 DNA polymerase (Fermentas), 0.05 U/μl Taq DNA polymerase (Kapa biosystems). Reactions were vortexed briefly to mix and incubated for 20 min at 25°C followed by 72°C for 20 min.

The reactions were placed on ice. A 5μl solution containing 1x Thermopol buffer (NEB), 10 times (fragments >700 bp) to 30 times (fragments <700 bp) the molar equivalent of the R.Block T7 adapter and 5 units of T4 DNA ligase (Fermentas) was added directly to each reaction. Reactions were vortexed to mix and incubated for 60 min at 22°C and for 15 min at 65°C. Similar reactions were pooled and mixed by vortexing. Samples were stored for no longer than 24 hours over night.

Reactions were fractionated on an LE agarose gel stained with 1x Cyber Green. Using a Dark Reader transillumiinator, gel slices containing fragments in the range of 800bp to 1200bp (Illumina sequencing) or 1200bp to 1600bp (454 sequencing) were excised. DNA fragments were recovered using Qiagen gel extraction columns and eluted in 50μl 5 mM Tris HCl pH 8.5.

**LMPCR of the template fragment library**

50μl PCRs contained 1x LongAmp buffer (NEB), 1pmol/μl of each LMPCR primer, 1 μg/μl Ultra Pure BSA (Ambion), 0.3mM dNTPs, 0.1U/μl LongAmp DNA polymerase (NEB) and 20μl of the purified ligated gDNA fragments.

PCRs were cycled as follows: 10x to 16x 95°C for 2 min, (95°C for 30 sec, 60°C for 30 sec, 72°C for 1 min to 1.5 min) 72°C for 5 min then held at 15°C.

PCRs were purified using MinElute columns (Qiagen) using the standard operating procedure, with the following exceptions: All centrifugations were performed at 16000 RCF. Elution buffer (EB - lOmM Tris HCl pH 8.5) was heated to 70°C. 10 μl of heated EB was added directly to each column followed by 5 min incubation at 70°C. The eluate was recovered by centrifugation. A further 10 μl of pre-heated EB was added to each column, incubated for 1 min at 70°C and the eluate recovered by centrifugation. Following purification, all eluates were pooled and vortexed. Eluted samples were stored at -20°C.

**Assessment of fragment size and quantification**

Fragment size and linker carry over were assessed using a DNA 7500 chip for the Bioanalyser 2100 (Agilent). The majority of fragments ranged from 800 bp to 1200bp for Illumina NGS fragment libraries and 1200 to 1600 bp for Roche 454 NGS fragment libraries.

Each library was quantified using a NanoDrop spectrophotometer (Thermo).

**Example 7 - Use of multi-biotinylated probes of Example 6 for in-solution target capture**

A series of in solution target capture experiments were undertaken to test the performance of the multi-biotinylated probe. In solution target capture workflow:

1. Fragment gDNA to the required size range (average fragment size 1kb for Illumina sequencing to 1.4 kb for Roche sequencing).

2. Ligate NGS platform specific linkers (Fragment library).

3. Perform 14 to 17 cycles of PCR to enrich correctly ligated DNA fragments

4. Hybridise ROI within the fragment library with the bait

5. Physically recover the bait and hybridised ROI by binding the bait's covalently linked biotin molecules with Streptavidin coated paramagnetic beads.

6. Wash the beads in a stringent wash buffer

7. Elute the captured ROI fragments with PCR

***Hybridisation according to the first to fourth aspects of the invention***

Hybridisation mixes contained: 0.75 $\mu$g to 1 $\mu$g of a gDNA fragment library (Average fragment size ~1 kb (Illumina MiSeq sequencing) or ~1.4 kb (Roche 454 GS FLX plus sequencing); 5 $\mu$g to 10 $\mu$g of a repetitive sequence blocker (as described in Example 8); 0 to 33 pmol/ul of oligonucleotides complementary to the library linkers (library blocking oligos); 1x Superase. IN RNase inhibitor; and 0.08 $\mu$M (~2500 individual probe sequences) to 0.13 $\mu$M (~16,000 individual probe sequences) of multi-biotinylated probe were diluted to 35$\mu$l in a proprietary hybridisation buffer. (0.02% Ficol, 0.04% PVP, 45mM Tris-HCl 11mM Ammonium Sulphate, 20mM MgCl$_2$, 6.8mM 2-Mercapthoethanol and 4.4mM EDTA. pH 8.5)

The hybridisation mixes were: incubated at 95°C for 2 min; cooled at a rate of 1°C every 10 sec to 10°C above a predefined optimal annealing temperature; step-down incubated for 60 sec at every °C above the optimal annealing temperature and cooled at a rate of 1°C every 10 sec between each °C; and incubated at the optimal annealing temperature for 24 hours.

A schematic representation of the hybridised target DNA with multiple non-overlapping multi-biotinylated probes is shown in Figure 4.

Referring to Figure 4, a hybridised target DNA of the second aspect of the invention is shown. The target DNA sequence (4) has been hybridised to a plurality of probes (6). The probes (6) are arranged such that they extend towards both flanks of the target DNA sequence (4).

Referring now to Figure 5, a probe or probe (6) of the third aspect of the invention, which can be used to form the hybridised DNA sequence (2) of Figure 4, is comprised of a probe DNA sequence (8) consisting of approximately 100 bases. The fragment (8) includes a plurality of biotin labels (10), spaced along the fragment (8). The fragment (8) includes a non-targeting end (14), which includes three biotin labels, one of which is a terminal biotin (12), connected within five bases of the non-targeting (14) end.

**Example 8 - Use of multi-biotinylated probes of Example 6 for on-surface target capture**

***Binding***

MyOne Streptavidin T1 paramagnetic dynabeads (Invitrogen) (1 mg) were washed twice in the proprietary hybridisation buffer (as defined in Example 7) either containing or not containing a nucleotide based blocking agent (R.block or DNA based).

The dynabeads were then re-suspended in 20 $\mu$l to 65 $\mu$l of the hybridisation buffer and incubated at 55°C for 30 min prior to heating to the pre-defined optimal annealing temperature.

Hybridisation mixes were then transferred to the binding solution, mixed with gentle pipetting and incubated at the optimal annealing temperature for 20 min.

*Washing*

Following hybridisation, the dynabeads were concentrated, re-suspended in 150 μl of a pre-heated proprietary wash buffer and incubated at a predefined washing temperature for 5 min. This was repeated once.

The dynabeads were concentrated, re-suspended in hybridisation buffer supplemented with 5 U of Hybridase thermostable RNase H (Epicentre) (total volume 50μl); incubated at 55°C for 30 min, and finally incubated at the pre-defined wash temperature for 5min.

The dynabeads were concentrated, re-suspended in 150 μl of a pre-heated proprietary wash buffer (50mM HEPES, 0.04% PVP, lOmM $MgCl_2$, 6.8mM 2-MercaptoEthanol. pH 8.5) and incubated at a predefined washing temperature for 5min.

The dynabeads were concentrated, re-suspended in 50μl lOmM Tris HCl (pH 8.5).

*Analysis*

Samples were eluted from the bead-captured probes by PCR prior to purification and NGS analysis using the Roche 454 GS FLX plus sequencing platform or the Illumina MiSeq sequencing platform.

*Enrichment power*

Enrichment power (EP) is a measurement of how well a target capture method performs.

Firstly, the ratio of NGS reads that overlap the targeted region over reads that do not overlap the target is calculated (fr).

Secondly, the fraction of the genome that is targeted is calculated (ft) EP can then be calculated. EP = fr ÷ ft.

**Results**

EP of 2000 to 3000 fold was achieved.

In all cases 90-95% of the target was recovered at a depth ≥20% of the average per base read depth, with ~80% recovered at ≥50% of the average.

**Example 9 - Preparation of RNA transcription products of DNA fragments for use in methods the invention and preparation of target DNA**

Eukaryotic gDNA was randomly fragmented to a range of sizes between 100 bp and 9000 bp to suit different applications. Adapters containing a T7 RNA polymerase promoter, or any other RNA polymerase promoter, were annealed to the fragmented DNAs, including Cot-1 DNA and repetitive sequence rich DNA from other eukaryotes.

The adapter ligated DNA fragments were either amplified by PCR prior to transcription to increase yield, or transcribed without amplification.

The fragments were transcribed from the promoter by T7 RNA polymerase, or any other RNA polymerase if the adapter contained a promoter other than the T7 promoter.

Following transcription, DNase I was used to remove contaminating DNA. Following DNase I treatment, Proteinase K was used to remove contaminating DNase and RNase. The RNA product was then purified and protected by the addition of a temperature reversible RNase inhibitor (SUPERase .IN - Ambion) or any other suitable RNase inhibitor.

The resultant product of the invention will hereinafter be called "R.Block".

Three R.Block types were produced using the above methods, namely:

1. R.Block-Hg (from Human gDNA fragmented to ~350 bp on average)
2. R.Block-Hc (from Human Cot-1 DNA)
3. R.Block-Sg (from Salmon gDNA fragmented to >800 bp)

**Preparation of DNA sample for R. block production**

A sample of DNA similar to the source of DNA for ultimate enrichment must be obtained. For example, if target enrichment of a human genomic DNA sample is required, either extract human genomic DNA from an un-related donor or purchase the DNA from a trusted supplier. The desired DNA was extracted according to standard procedures, and dissolved in 10 mM Tris HCl (pH 8.5).

**gDNA fragmentation**

The following method describes fragmentation using a Bioruptor (RTM) sonicator. Note: Other DNA fragmentation options may be implemented, for example the Covaris system (Covaris), nebulisation (Roche), or by NEBNext dsDNA Fragmentase (NEB).

The gDNA was diluted in 10 mM Tris HCl (pH 8.5) to a concentration of 20 ng/$\mu$l. 110 $\mu$l of the diluted DNA was aliquoted into separate 1.5 ml sonication tubes (Diagenode), vortexed and centrifuged briefly prior to incubation on ice until the Bioruptor (Diagenode) was prepared.

To prepare the Bioruptor(RTM), the shearing bath was chilled for 30 min with water containing an ~0.5 cm layer of crushed ice. Following preparation, the aliquots of gDNA were placed into the Bioruptor's (RTM) sample cradle and device assembled according to the manufacturers guidelines.

The samples were sonicated as follows:

| | |
|---|---|
| Power setting | Low |
| Sonication cycle | 15 to 30 sec on followed by 90 sec off |
| Number of cycles | 5-25 (dependant on the required level of sonication) |

Following sonication the fragmented DNAs were pooled and stored at -20°C.

**Small fragment removal purification of the DNA fragments**

Aliquots of the pooled sheared gDNA were purified using 1.2x to 1.8x AmpureXP beads (Beckman Coulter), dependant on the required fragment size, according to the manufacturers standard operating procedure. Finally the DNA was eluted in 10 mM Tris HCl (pH 8.5) with incubation at 65°C for 5 min prior to removal of the magnetic beads. Purified sheared gDNAs were quantified using a NanoDrop spectrophotometer and the fragment size determined using a DNA 7500 chip for the bioanalyser 2100 (Agilent). Purified sheared gDNA was stored at -20°C.

**Fragment polishing, dA tailing and linker ligation to produce a raw R.Block template library**

25 $\mu$1 reactions were prepared on ice containing 500ng to 1000ng of fragmented gDNA, 1x Thermopol buffer (NEB), 2% PEG 4000 (Fermentas) 1.0 mM ATP (Thermo), 0.4 mM dNTPs (Promega) 0.4 U/$\mu$l T4 polynucleotide kinase (Fermentas), 0.1 U/$\mu$l T4 DNA polymerase (Fermentas), 0.05 U/$\mu$l Taq DNA polymerase (Kapa biosystems). Reactions were vortexed briefly to mix and incubated for 20 min at 25°C followed by 72°C for 20 min.

The reactions were placed on ice. A 5$\mu$l solution containing 1x Thermopol buffer (NEB), 10 times (fragments >700 bp) to 30 times (fragments <700 bp) the molar equivalent of the R.Block T7 adapter and 5 units of T4 DNA ligase (Fermentas) was added directly to each reaction. Reactions were vortexed to mix and incubated for 60 min at 22°C and for 15 min at 65°C. Similar reactions were pooled and mixed by vortexing. Samples were stored for no longer than 24 hours over night.

Reactions were purified using 1.8x AmpureXP beads (Beckman Coulter), according to the manufacturer's standard operating procedure. Finally the DNA was eluted in 25$\mu$l 10 mM Tris HCl (pH 8.5) with incubation at 65°C for 5 min prior to removal of the magnetic beads. Fragment size and linker carry over was assessed using a DNA high sensitivity chip for the bioanalyser 2100 (Agilent). Purified sheared gDNA was stored at -20°C.

**LMPCR of the R.Block template library**

50$\mu$l PCRs contained 1x LongAmp buffer (NEB), 1pmol/ul or each LMPCR primer, 1 $\mu$g/$\mu$l Ultra Pure BSA (Ambion), 0.3mM dNTPs, 0.1U/$\mu$l LongAmp DNA polymerase (NEB) and 20$\mu$l of the purified ligated gDNA fragments.

PCRs were cycled as follows: 10x to 16x 95°C for 2 min, (95°C for 30 sec, 60°C for 30 sec, 72°C for 1 min to 1.5 min) 72°C for 5 min then held at 15°C.

Several pooled PCRs were purified using MinElute columns (Qiagen) using the standard operating procedure, with the following exceptions: All centrifugations were performed at 16000 RCF. Elution buffer (EB - lOmM Tris HCl pH 8.5) was heated to 70°C. 10 $\mu$l of heated EB was added directly to each column followed by 5 min incubation at 70°C. The eluate was recovered by centrifugation. A further 10 $\mu$l of pre-heated EB was added to each column, incubated for 1 min at 70°C and the eluate recovered by centrifugation. Following purification, all eluates were pooled and vortexed. Eluted samples were stored at - 20°C.

**Assessment of fragment size and quantification of the R.Block template library**

Fragment size and linker carry over were assessed using a DNA 7500 chip for the bioanalyser 2100 (Agilent). The majority of fragments ranged from 100 bp to 500 bp for R.Block-Hc (derived from human Cot-1 DNA), 200 to 700 bp for R.Block-Hg (genomic sequence derived from human DNA) and >700 bp for R.Block-Sg (genomic sequence derived from Salmon DNA).

Each library was quantified using a NanoDrop spectrophotometer (Thermo).

**Transcription of the R.block template library**

25 µl Transcription reactions contained 1 µg of an R.Block template library, 1x RNAMaxx transcription buffer Agilent) 4mM of each rNTP, 30 mM DTT (Agilent), 0.015 U/µl Yeast inorganic Pyrophosphatase (Agilent), 1 U/µl SUPERase .IN (Ambion) and 8 U/µl T7 RNA polymerase (Agilent). Reactions were incubated for 2 hours at 37°C.

To stop the reactions 1µl Turbo DNase (2 U/µl) was added to each separate reaction and incubated for 30 min at 37°C.

A mixture of 6 µl RNAMaxx 5x transcription buffer, 2.5 µl SUPERase. IN, 23.5 µl 5 M Urea and 3 µl proteinase K (recombinant) (Thermo) was added to each reaction. Reactions were incubated for 30 min at 37°C. Reactions were held on ice and were not stored until purified.

**Purification of the R.Block**

Sufficient MicroBioSpin p6 columns (BioRad) were warmed to room temperature such that 75 µl of un-purified probe library could be passed through each column. The probe library was purified according to the manufacturer's standard operating procedure. Following purification, eluates were pooled prior to the addition of one 20th the volume of SUPERase. IN (Ambion). R.Blocks were gently mixed and stored at -80°C.

**Assessing the R.Block fragment size and concentration**

R.Block Fragment size and linker carry over were assessed using an RNA 6000 nano chip for the bioanalyser 2100 (Agilent). A high quality R.block had the following features: The majority of fragments ranged from >100 nt for R.Block-Hc, >200 nt for R.Block-Hg and >800 nt for R.Block-Sg (genomic sequence derived from Salmon DNA); >80 µg total Mass of R.Block per transcription; Very little primer or linker contamination.

R.Blocks were stored at -80°C.

**Example 10 - Optimised preparation of R.Block products with multi-biotinylated probes and optimised hTE hybridisation protocol for network blocking and surface blocking**

**Materials and Methods**

*Probe sequence design*

A custom oligonucleotide design software (Lancaster, O. et al., Unpublished) was used to design non-overlapping (minimum gap =5nt) nucleotide sequences (average 60nt). Probes were designed to have a tm between 65°C and 75°C, and were extended or contracted by up to 10nt to fit within the tm range. No Probes were placed within 10bp of repetitive sequences. Each probe was permitted to match the genome ≤5 times. The software then calculated the average Tm of all identified sequences. Subsequently, for each kb of targeted sequence, the 10 sequences that most closely matched the average Tm were selected. The remaining sequences were discarded.

The software output the probe sequences as a FASTA file (9) which was submitted to Mycroarray (Mycroarray MI USA). We developed a custom perl script to add primer annealing sites to each sequence (5' CTGGCAGACGAGAGGCAGTG/genomic sequence/GTAGACCTCACCAGCGACGC 3'). The resulting FASTA file was then converted, using the same custom perl script) into a tab delimited text based table.

The template probe pool, that contained all the sequences contained in the tab delimited text based table, was was synthesised so that each individual probe was synthesised at seven different loci on a microarray (Mycroarray). Following synthesis, the probes were harvested and lyophilised by the manufacturer prior to shipping. The probes were reconstituted in lOmM Tris Hcl (pH 8) (Qiagen) to a stock concentration of ~10ng/µl. Working concentrations of ~10pg/µl were prepared by serially diluting the stock probe pool with Tris Hcl (pH 8).

*Generating multi-biotinylated DNA hTE probes*

50µl PCRs contained 1x Q5 reaction Buffer (NEB), 1.5µM ProAmpF04E (5' phosphate-CTGGCAGACGAGAGGCAGTG 3'), 1.5M ProAmpR01 (5' biotin-TEG-GCGTCGCTGGTGAGGTCTAC 3'), 300µM dTTP, 300µM dATP, 300µM dGTP, 105µM dCTP (Promega), 195µM Biotin-16-Aminoallyl-2'-dCTP (Trilink BioTechnologies), 1U Thermostable Inorganic Pyrophosphatase (NEB), 0.5U Q5 DNA polymerase (NEB), 10pg-20pg template probe pool (Mycroarray). Reactions were sealed with a heat sealable PCR film or PCR strip-caps (Thermo fisher Scientific). The reactions were cycled as follows: 98°C for 2min, 17x (98°C for 15sec, 72°C for 25sec), 72°C for 1 minute then held at 15°C.

100 PCRs were pooled and vortexed. 200µl aliquots of the pooled PCRs were purified using MinElute columns (Qiagen) using the standard operating procedure, with the following exceptions: All centrifugations were performed at 16000 RCF. Elution buffer (EB - 10mM Tris HCl pH 8.5) was heated to 70°C. 10µl of heated EB was added directly to each column and incubated at 70°C for 5 min. The eluate was recovered by centrifugation for 1 min. A further 10 µl of pre-heated EB was added to each column, incubated for 5 min at 70°C and the eluate recovered by centrifugation for 1 min. Following purification, all eluates were pooled and vortexed.

A bulk reaction was prepared on ice such that every 20µl contained 2µg amplified complex pool, 1x Terminal Transferase buffer (NEB), 1x CoCl$_2$ (NEB), 2.5U *Bts*I, 4µg BSA (NEB) and 500µM ddATP (Trilink). The reaction was mixed by vortexing and incubated for 30 min at 55°C. The reaction was incubated on ice for 5 min.

3µl of a mixture containing 50U of Terminal Transferase (NEB) in 1x Terminal Transferase buffer (NEB) was added per 20µl of the reaction. The reaction was vortexed to mix and incubated for 60 min at 37°C. The reaction was incubated on ice for 5 min.

3µl of a mixture containing 12.5U of λ exonuclease (NEB) in 1x Terminal Transferase buffer (NEB) was added per 20µl of the initial bulk reaction volume. The reaction was vortexed to mix and incubated for 20 min at 37°C and 20 min at 80°C.

Sufficient MicroBioSpin p6 columns (BioRad) were warmed to room temperature such that 75µl of un-purified probe library could be passed through each column. The probe library was purified according to the manufacturer's standard operating procedure. Following purification, the eluates were pooled and gently vortexed.

The purified probe library was analysed using an RNA 6000 nano chip for the Bioanalyser 1100 (Agilent) and quantified using a NanoDrop spectrophotometer (Thermo) An ideal hTE capture probe library had a concentration of ≥50 ng/µl, an OD 260:280 of 1.7 - 2.0 and had an average fragment size of ~150nt (the fragment size is >100nt due to the presence of biotin molecules retarding migration through the gel matrix).

*Fragmentation of human gDNA for fragment library preparation*

Human gDNA was diluted in lOmM Tris HCl (pH 8.5) to a concentration of 20ng/µl 110µl of the diluted DNA was aliquoted into separate 1.5ml sonication tubes (Diagenode), vortexed and centrifuged briefly prior to incubation on ice until the Bioruptor (Diagenode) was prepared. The Bioruptor's shearing bath was chilled for 30 min with water containing an ~0.5 cm layer of crushed ice. Following preparation, the aliquots of gDNA were placed into the Bioruptor's sample cradle and device assembled according to the manufacturers guidelines. The samples were sonicated as follows: Power setting Low, Sonication cycle 15sec on followed by 90sec off for 14 cycles to 16 cycles.

Aliquots of the pooled sheared gDNA were purified using 0.8x AmpureXP beads (Beckman Coulter) according to the manufacturers standard operating procedure. The DNA was eluted in lOmM Tris HCl (pH 8.5) with incubation at 65°C for 5 min prior to removal of the magnetic beads. Purified sheared gDNAs were quantified using a NanoDrop spectrophotometer and the fragment size determined using a DNA 7500 chip for the bioanalyser 2100 (Agilent). An ideally fragmented library had an average fragment size of ~1200bp with >50% of fragments falling in the range of 1000 to 2000bp. Purified sheared gDNA was stored at -20°C.

*Preparation of human fragment libraries for Illumina MiSeq sequencing*

**End repair and ligation:** Illumina TruSeq adaptors were added to 2.5µg aliquots of fragmented human gDNA using the NEBNext DNA Library Prep Master Mix Set for Illumina sequencing (E6040) and the NEBNext Multiplex Oligos for Illumina sequencing (Index Primers Set 1) (E7335).

**Size selection:** Reactions were fractionated on a 1.0% LE agarose gel stained with 0.2mg/ml EtBr. Using a Dark Reader transilluminator (Clare Chemical Research), gel slices containing fragments in the range of 1000bp to 2000bp were excised. DNA fragments were recovered using Qiagen gel extraction columns and eluted in 22µl lOmM Tris HCl pH 8.5.

**Linker mediated PCR enrichment for ligated fragments:** For each ligated DNA library 4x 100µl PCRs contained 1x Q5 High-Fidelity 2X Master Mix, 1µM NEBNext Universal PCR Primer for Illumina, 1µM NEBNext Index Primer for Illumina and 5µl of ligated fragments.

PCRs were cycled as follows: to 98°C for 30sec, 8x to 10x (98°C for 10 sec, 65°C for 1min 15sec) 65°C for 1 min then held at 15°C. PCRs were pooled and purified using 0.5x AmpureXP beads (Beckman Coulter) according to the manufacturers standard operating procedure. Recovered fragment library DNA was eluted in 25$\mu$l lOmM Tris HCl (pH 8.5) with incubation at 65°C for 5 min prior to removal of the magnetic beads. Eluted samples were stored at -20°C.

**QC:** Fragment size and linker carry over were assessed using a DNA 7500 chip for the bioanalyser 2100 (Agilent). The average fragment size was ~1300bp. Each library was quantified using a NanoDrop spectrophotometer (Thermo Fisher Scientific).

## *Fragmentation of gDNA samples for R. Block production*

Human and salmon gDNA was diluted in lOmM Tris HCl (pH 8.5) to a concentration of 20 ng/$\mu$l. 110 $\mu$l of the diluted DNA was aliquoted into separate 1.5 ml sonication tubes (Diagenode), vortexed and centrifuged briefly prior to incubation on ice until the Bioruptor (RTM) (Diagenode) was prepared. The Bioruptor's (RTM) shearing bath was chilled for 30 min with water containing an ~0.5 cm layer of crushed ice. Following preparation, the aliquots of gDNA were placed into the Bioruptor's (RTM) sample cradle and device assembled according to the manufacturers guidelines. The samples were sonicated as follows: Power setting Low, 15 to 30 sec on followed by 90 sec off for 2cycles to 4 cycles for the Salmon gDNA and 22cycles to 24 cycles for the Human gDNA

Aliquots of the pooled sheared gDNA were purified using 1.8x AmpureXP beads (Beckman Coulter), dependant on the required fragment size, according to the manufacturers standard operating procedure. Finally the DNA was eluted in 10 mM Tris HCl (pH 8.5) with incubation at 65°C for 5 min prior to removal of the magnetic beads. Purified sheared gDNAs were quantified using a NanoDrop spectrophotometer and the fragment size determined using a DNA 7500 chip for the bioanalyser 2100 (Agilent). The average size for the human gDNA was ~500bp and for the salmon gDNA, 3000bp. Purified sheared gDNA was stored at -20°C.

## *Preparation of R.Block DNA template*

**End repair:** 25$\mu$l reactions were prepared on ice containing 1000ng of fragmented gDNA or or human Cot-1 DNA, 1x Fast Digest buffer (Fermentas), 1mM ATP (Thermo), 0.4mM dNTPs (Promega) 10U T4 polynucleotide kinase (Fermentas), 2.5U T4 DNA polymerase (Fermentas), 1.25U Taq DNA polymerase (Kapa biosystems). Reactions were vortexed briefly to mix and incubated for 20min at 25°C followed by incubation at 72°C for 20min.

**Ligation:** The reactions were placed on ice. 0.4$\mu$M R.Linker (5' CGACCGACTGCCACCTGCGCTAATAC-GACTCACTATAGGGCTAGTGCTTCGCATC CGA*A*G*T* 3'; 5' phosphate-CTTCGGATGCGAAGCACTAGGGCGTGCAGCCTGTGGC*A*G*C 3'; where * denote a phosphorothioate Bond) and 5U of T4 DNA ligase (Fermentas) was added directly to each reaction. Reactions were vortexed to mix and incubated for 20min at 25°.

**Linker removal:** Samples were purified using 1.8x Ampure XP beads. Recovered fragments were recovered in 50$\mu$l lOmM Tris HCl (pH 8).

**Linker mediated PCR enrichment for ligated fragments:** 100$\mu$l PCRs contained 1x FastStart high fidelity buffer (Roche), 1$\mu$M of each fragment library Linker Mediated PCR (LMPCR) primer (5' CGACCGACTGCCACCTGCGC 3'; 5' GCTGCCACAGGCTGCACGCC 3'), 2% DMSO (Sigma-Aldrich), 0.2mM dNTPs, 5U FastStart DNA polymerase blend (Roche) and 50$\mu$l of the ligated gDNA fragments. PCRs were cycled as follows: to 95°C for 10min, 12x (95°C for 30 sec, 64°C for 30 sec, 72°C for 3min) 72°C for 7min then held at 15°C. PCRs were purified using 1.8x AmpureXP beads (Beckman Coulter) according to the manufacturers standard operating procedure. Recovered fragment library DNA was eluted in 25$\mu$l lOmM Tris HCl (pH 8.5) with incubation at 65°C for 5 min prior to removal of the magnetic beads. Eluted samples were stored at -20°C.

**QC:** Fragment size and linker carry over were assessed using a DNA 7500 chip for the bioanalyser 2100 (Agilent). Each library was quantified using a NanoDrop spectrophotometer (Thermo Fisher Scientific).

## *Transcription of the R.Block DNA template library*

25$\mu$l Transcription reactions contained 1$\mu$g of an R.Block DNA template library (human gDNA, salmon gDNA and human Cot-1 DNA), 1x RNAMaxx transcription buffer Agilent) 4mM of each rNTP, 30mM Dithiothreitol (Agilent), 0.015 U/$\mu$l Yeast inorganic Pyrophosphatase (Agilent), 25 U SUPERase .IN (Ambion) and 200U T7 RNA polymerase (Agilent). Reactions were incubated for 2 hours at 37°C. To stop the reactions 2U Turbo DNase (Thermo Fisher Scientific) was added to each separate reaction and incubated for 30 min at 37°C.

A mixture of 1x RNAMaxx transcription buffer, 50U SUPERase. IN, 23.5 $\mu$l 3.35M Urea and 6mg proteinase K (recombinant, PCR grade) (Thermo Fisher Scientific) was added to each reaction. Reactions were incubated for 30 min at 37°C.

Sufficient MicroBioSpin p6 columns (BioRad) were warmed to room temperature such that 75 $\mu$l of un-purified

probe library could be passed through each column. The probe library was purified according to the manufacturer's standard operating procedure. Following purification, eluates were pooled prior to the addition of one 20th the volume of SUPERase. IN (Ambion).

The R.Block products produced are hereinafter labelled R.Block -Hg (derived from human genome DNA), R.Block -Hc (derived from human Cot-1 DNA) and R.Block -Sg (derived from salmon genome DNA).

R.Block Fragment size and linker carry over were assessed using an RNA 6000 nano chip for the bioanalyser 2100 (Agilent). A high quality R.block had the following features: The majority of fragments ranged from >200 nt for R.Block-Hg (derived from human gDNA) and R.Block -Hc (derived from human Cot-1 DNA) and >800 nt for R.Block -Sg (derived from salmon gDNA); >80 $\mu$g total Mass of R.Block per transcription; Very little primer or linker contamination. R.Blocks were stored at -80°C.

### *OptimisedIn-solution hTE protocol*

### Hybridisation

30$\mu$l hybridisation mixes contained: 1x hybridisation buffer (0.02% Ficol, 0.04% PVP, 45mM Tris-HCl 11mM Ammonium Sulphate, 20mM MgCl$_2$, 6.8mM 2-Mercapthoethanol and 4.4mM EDTA. pH 8.5), 0.5$\mu$g DNA fragment library (above), R.Block (Hg, Hc or Sg) 10$\mu$g (unless stated otherwise), 30U Superase. IN RNase inhibitor and 60ng multi-biotinylated probe (as above).

The hybridisation mixes were: incubated at 98°C for 2 min; cooled at a rate of 1°C per second to 72°C; step-down incubated for 60 sec at 1°C intervals, cooled at a rate of 1°C per second between each interval; and incubated at 62°C for 24 hours.

In other examples the incubation steps may be performed at a temperature in the range of 50°C to 80°C, depending on the molecules hybridised, as will be determined by the skilled person.

### Binding

0.75mg MyOne Streptavidin C1 paramagnetic dynabeads (Invitrogen) were washed twice in 100$\mu$l 1x hybridisation buffer. The dynabeads were then re-suspended in 20$\mu$l 1x hybridisation buffer supplemented with 10$\mu$g of R.Block or other blocker (unless stated in the results). The resulting binding solutions were incubated at 55°C for 30 min prior to heating to 62°C. The hybridisation mixes were then transferred to the binding solutions, mixed with gentle pipetting and incubated at 62°C for 20 min.

In other examples the binding steps may be performed at a temperature in the range of 50°C to 80°C, depending on the molecules hybridised, as will be determined by the skilled person

### Washing

Following hybridisation, the dynabeads were concentrated, and the hybridisation solution removed. The samples were returned to 62°C prior re-suspension of the dynabeads in 150$\mu$l of pre-warmed (62°C) 1x wash solution (50mM HEPES, 0.04% PVP, lOmM MgCl$_2$, 6.8mM 2-MercaptoEthanol. pH 8.5). The samples were incubated at 62°C for 5 min.

The dynabeads were concentrated, and the wash solution removed. The samples were returned to 62°C prior re-suspension of the dynabeads in 50$\mu$l of 1x hybridisation buffer supplemented with 5U of Hybridase Thermostable RNase H (Epicentre). The samples were incubated at 62°C for 15min.

The dynabeads were concentrated and the RNase solution was removed. The beads were washed once more (as above) in 150$\mu$l pre-heated wash solution, incubated at 62°C for 5 min.

The dynabeads were concentrated and the wash solution was removed. The dynabeads were re-suspended, at room temperature, in 50$\mu$l lOmM Tris HCl (pH 8.5).

In other examples, washing steps may be performed at a temperature in the range of 50°C to 80°C, depending on the molecules hybridised, as will be determined by the skilled person.

### LMPCR elution of the captured DNA library fragments for MiSeq sequencing

4x 100$\mu$l PCRs contained 1x Q5 PCR master-mix (NEB), 2$\mu$M of each library amplification primer (5' AATGATACGGCGACCACCGAG 3'; 5' CAAGCAGAAGACGGCATACGAG 3') and 10$\mu$l of the bead bound captured DNA library. PCRs were cycled as follows: to 98°C for 30sec, 10x (98°C for 30 sec, 65°C for 1.5min) 65°C for 5min then held at 15°C.

PCRs were purified using 0.5x AmpureXP beads (Beckman Coulter) according to the manufacturers standard operating procedure. Recovered fragment library DNA was eluted in 50$\mu$l lOmM Tris HCl (pH 8.5) with incubation at 65°C for 5 min prior to removal of the magnetic beads. Eluted samples were stored at -20°C.

*Next generation sequencing of the enriched DNA libraries*

All libraries had been prepared with linkers containing multiplex identifier sequences to allow sample pooling prior to sequencing. Illumina MiSeq sample pooling and sequencing was performed at the University of Leicester Genomics Service Facility (NUCLEUS). Sequencing was performed using the MiSeq Reagent Kit v3 (2x300nt) sequencing chemistry (Illumina).

*Probe sequence file formats*

Probe sequences were aligned to the human genome (GRCh37.p13 http://www.ncbi.nlm.nih.gov/projects/genome/assembly/grc/human/data/) using the Bowtie 2 alignment algorithm (10), specifying the -f flag to state that the probe sequences were in the FASTA format (above) and the -S flag to indicate that the output should be written into files in the SAM format. The alignments, that were generated by Bowtie 2, in the SAM format were converted into a sorted and indexed BAM format using SAMtools (11). Finally, the bamToBed function of BEDtools (12) was used to tabulate the coordinates of each probe sequence in the BED format: chromosome number; start coordinates; and end coordinates.

*NGS sequence file formats*

FASTQ files were returned as standard from Illumina MiSeq sequencing. The Bowtie2 alignment tool was used to align the NGS sequences to the human genome (GRCh37.p13). The -q flag was used to indicate that the sequences were in the FASTQ format, the -1 and -2 flags indicated that the NGS data comprised sequence pairs and the -S flag indicated that the output should be written into files in the SAM format. The output SAM files were converted to into sorted and indexed BAM files using SAMtools. Copies of the BAM files were made, and from these copied files, sequence duplicates were removed using the MarkDuplicates tool (Remove_Duplicates=True) of the Picard tool set (http://broadinstitute.github.io/picard/). HTE quality metrics were calculated using the Target Enrichment Quality Control (TEQC) (13) library for the R statistical package (14) (Results).

The raw BAM files were imported into TEQC. TEQC was used to filter out valid NGS sequence pairs (read-pairs) with the maximum distance permitted between reads paired sequences set to 5kb.

The potential advantages of blocking with an R.Block molecule (such as produced by the methods of the invention, as exemplified in Examples 9 and 10) are:

1. R.Block generated from highly repetitive DNA, e.g. Cot-1 DNA, mask interspersed repetitive sequences making them unavailable for non-specific hybridisation.

2. R.Block generated from gDNA mask both interspersed repetitive sequences and non-repetitive sequences making them unavailable for non-specific hybridisation to probes. Note: The probe concentration needs to be sufficient such that it out-competes R.Block : target hybridisation.

3. Whole genome masking by R.Block generated from gDNA reduces networking between regions not found in Cot-1 DNA, e.g. Segmental Duplications and self-chain alignments.

4. RNA : DNA duplexes are more stable than DNA : DNA duplexes. Captured gDNA fragments containing repetitive sequences are more likely to hybridise to R.Block rather than other captured gDNA fragments.

5. RNA: DNA duplexes are more stable than DNA : DNA duplexes, so R.Block is more resistant to stringent conditions than equivalent DNA blockers.

6. A range of RNase species with differing properties can be used to break down the R.Block. Additional washing will remove an additional fraction of off-target fragments. This makes the R.Block versatile and useful for a range of additional applications.

7. R.Block can potentially be used in conjunction with RNA based probes and probes so long as RNase $I_f$ (NEB) is used to break down the R.Block. RNase $I_f$ preferentially cleaves single-stranded RNA rather than RNA : RNA or RNA : DNA duplexes. It may therefore be possible to optimise and use an R.Blocker in a target capture system based on RNA probes e.g. SureSelect (Agilent).

**Example 11** - **Use of R-Block products in a method of the invention for interspersed repeat DNA blocking**

A series of investigations to determine whether R.Block effectively blocks network formation via interspersed repeat DNA were performed.

The R.Block Products and multi-biotinlyated probes used, were manufactured according to the process described in Example 10. The investigation was an in solution target DNA capture.

The following R.Block preparations made according to the method of Example 10 were tested as blocking agents to block network binding of interspersed repetitive DNA sequences:

1. 5 μg R.Block based on Cot-1 DNA ("R.Block-Hc", h. Cot-1 in Figure 6)
2. 10 μg R.Block based on Cot-1 DNA
3. 5 μg R.Block based on human gDNA ("R.Block Hg", h.g DNA in Figure 6
4. 10 μg R.Block based on human gDNA

Hybridisation was performed according to the optimised hTE procedure of Example 10.

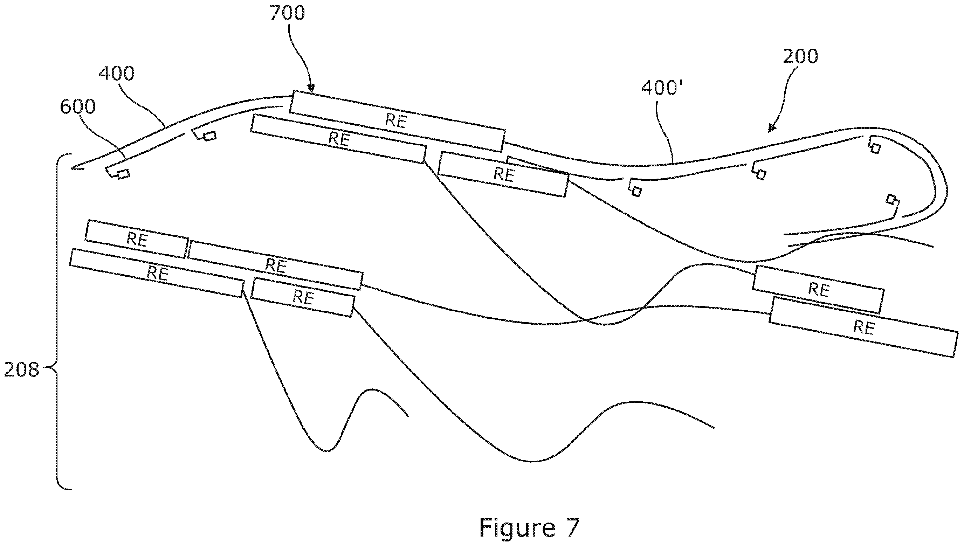

Figure 7, illustrates a representation of the of hybridised target DNA sequence (200), comprising target DNA sequence fragments (400, 400') flanking a repetitive element (700). The target DNA fragments (400, 400') are hybridised with a plurality of probes (600), the probes (600) being as described herein above . The hybridised target DNA sequence is part of a repetitive element network (208), formed by annealing of repetitive elements located on the target DNA sequence (200), probe (600), and non-specific DNA sequences. The network (208) can be destroyed by the addition of the R.Block products of this example and the invention during hybridisation mix incubation. Addition of R-Block to the network (208) destroys the network by destroying the repetitive element-repetitive element annealing; the majority of the network (208) is disrupted and this leaves specific hybridised target DNA sequences (200).

### Results

In brief it was found that R.Block based on human gDNA was a more effective network blocker than R.Block based on Cot-1 DNA. 10 μg of R.Block performed more effectively than 5 μg of R.Block, as shown in Figure 6.

### Example 12 - Use of R.Block products for blocking a surface

#### *Hybridisation*

For this investigation, "hybridisation mixes" contained: 1 μg of a gDNA fragment library of Example 6 (Average fragment size ~1.2kb); one blocker selected from:

1. 5 μg human Cot-1 DNA.
2. 2.5 μg Salmon gDNA and 2.5 μg human Cot-1 DNA.
3. 5 μg R.Block-Hg
4. 5 μg R.Block-Sg
5. No blocking agent

33 pmol/ul of oligonucleotides complementary to the library linkers (library blocking oligos); and 1x Superase. IN RNase inhibitor, diluted to 30 μl in a proprietary hybridization buffer (containing 0.02% Ficol, 0.04% PVP, 45mM Tris-HCl 11mM Ammonium Sulphate, 20mM $MgCl_2$, 6.8mM 2-Mercaptoethanol and 4.4mM EDTA. pH 8.5) The hybridisation mixes did not contain any biotinylated probe and the R.Block products were thus made according to a similar process described in Example 9.

The hybridisation mixes were: incubated at 95°C for 2 min; cooled at a rate of 1°C every 10 sec to 10°C above a pre-defined optimal annealing temperature; step-down incubated for 30 sec at every °C above the optimal annealing temperature and cooled at a rate of 1°C every 10 sec between each °C; and incubated at the optimal annealing temperature for 24 hours.

#### *Binding*

1 mg of streptavidin coated paramagnetic dynabeads was washed twice in the proprietary hybridisation buffer.

Two different dynabeads were used for this investigation.

1. MyOne streptavidin T1 (Invitrogen) was pre-coated with BSA by the manufacturer.
2. MyOne streptavidin C1 (Invitrogen) were un-coated

It was found that MyOne Streptavidin T1 tended to clump at temperatures ≥60°C, so its use was stopped.

Washing of the MyOne streptavidin C1 dynabeads at 65°C reduced non-specific interaction between the dynabead surfaces and gDNA fragments better than washing at 55°C.

The dynabeads were then re-suspended in the hybridisation buffer and one of the following surface blocking agents was added:

1. 5 µg human Cot-1 DNA.
2. 2.5 µg Salmon gDNA and 2.5 µg human Cot-1 DNA.
3. 5 µg R.Block-Hg.
4. 5 µg R.block-Hc.

The surface blocking agents act to mask or block repetitive sequence binding to the dynabeads.
These binding mixes were incubated at 55°C for 30 min prior to heating to the pre-defined optimal annealing temperature.
Hybridisation mixes were then transferred to the binding solution, mixed with gentle pipetting and incubated at the optimal annealing temperature for 20min.

*Washing*

Following hybridisation, the dynabeads were concentrated, re-suspended in a wash buffer (50mM HEPES, 0.04% PVP, IOmM MgCl$_2$, 6.8mM 2-MercapthoEthanol. pH 8.5). and incubated at a predefined washing temperature for 5min.
The dynabeads were concentrated, re-suspended in: 1x RNase I$_f$ buffer (NEB); 50 U RNase If (NEB) (unless stated); and 1% Triton X-100 (Fluka) (total volume 50 µl); incubated at 37°C for 15 min, and finally incubated at the predefined wash temperature for 5 min.
The dynabeads were again concentrated, re-suspended in a proprietary wash buffer and incubated at a pre-defined washing temperature for 5 min.
Finally, the dynabeads were concentrated, re-suspended in 50 µl 10 mM Tris HCl (pH8.5).

*qPCR*

Control curve: An aliquot of the fragment library used for this investigation was initially diluted to 1000 ng/µl. An aliquot was further diluted to 500 ng/µl. These samples were serially diluted by a factor of 1 in 10 to cover the range from 1 ng/µl to 0.0005 ng/µl.
Primary PCR: Duplicate 25 µl PCRs contained 1x Maxima SYBR Green hot start qPCR master mix (Maxima HS) (Thermo), 0.96 µM Rapid A PCR primer, 0.96 µM Rapid B PCR primer and 10 µl vortexed test dynabeads (see above) or control DNA. PCRs were heated to 95°C for 10 min followed by 7 cycles of (95°C for 30sec; 64°C for 30 sec and 72°C for 3min). Finally PCRs were incubated at 72°C for 5min.
Secondary PCR: 25 µl PCRs contained 1xMaxima HS, 0.96 µM Rapid A PCR primer, 0.96 µM Rapid B PCR primer and 1 µl primary PCR following magnetic concentration of the beads (concentration not required for the control PCRs). PCRs were performed on the Light Cycler 480 (Roche). PCRs were heated to 95°C for 10 min followed by 30 cycles of (95°C for 30sec; 64°C for 30; 72°C for 3min; and imaging).

*Analysis of the qPCR data*

A standard curve was plotted for the control series. The mass of gDNA library bound to each 0.2 mg of dynabeads was determined relative to the standard curve. The recovered mass was used to calculate the percentage of library fragment recovery caused by interactions with the dynabeads surface.
Results Cot-1DNA offered no significant reduction in bead surface to DNA fragment interaction when compared to un-blocked beads. Background recovery in both cases was >0.3%.
Samples containing combinations of Salmon gDNA with Cot-IDNA, R.Block -Hc or R.Block-Hg reduced background DNA fragment recovery to <0.01%. 5 µg and 10 µg of R.Block based on salmon gDNA ("R.Block-Sg") was also tested. Results indicated that the efficacy of blocking non-specific capture of DNA was, in order, R.Block -Sg > R.Block -Hg > R.Block -Hc.

**Example 13** - **Use of R.Block products vs DNA based blockers for network blocking and surface blocking**

For this investigation, the hybridisation and binding protocol of Example 10 was used,with varying combinations of blocking agent, one for use in the hybridisation mix (as network blocker during the hybridisation step of Example 10) and the other in the surface blocking mix (binding mix during the binding step of Example 10).

1. 10 µg Cot-1 DNA in the hybridisation mix ("network blocker"), 10 µg Cot-1 DNA in the blocking mix ("surface blocker").
2. 10 µg Cot-1 DNA in the hybridisation mix, 10 µg salmon gDNA in the blocking mix.

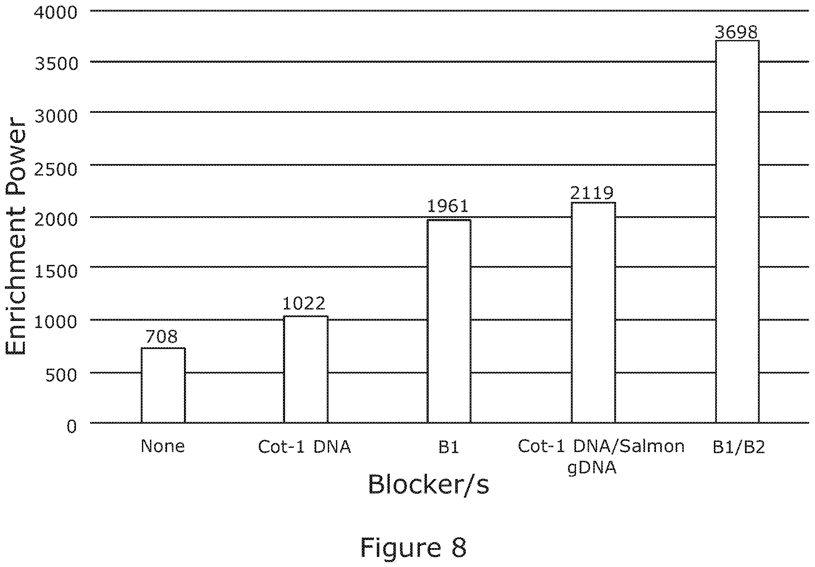

3. 10 μg R.Block-Hg in the hybridisation mix, 10 μg R.Block-Hg in the blocking mix (B1 in Figure 8).
4. 10 μg R.Block-Hg in the hybridisation mix, 10 μg salmon gDNA in the blocking mix (B1/B2 in Figure 8).
5. 10 μg R.Block-Hg in the hybridisation mix, 10 R.Block-Sg in the blocking mix.
6. 10 μg R.Block-Hg in the hybridisation mix, 10 R.Block-Hc in the blocking mix.
7. No blocker in either the hybridisation mix or the blocking mix.

The target DNA comprised 1 μg of a gDNA fragment library (average fragment size ~1 kb). MyOne Streptavidin C1 paramagnetic dynabeads were used instead of MyOne Streptavidin T1 (Invitrogen). The dynabeads were washed three times in 1x hybridisation buffer at room temperature. Finally the dynabeads were re-suspended in 20 μl to 65 μl of 1x hybridisation buffer containing 1 U/μl SUPERase .IN (Ambion) and 5 μg of the relevant blocking agent. This was incubated at 55°C for 30 min prior to being heated to a predetermined binding temperature and the addition of the hybridisation mix. Next generation sequencing was performed on the Illumina MiSeq platform.

**Results**

The results for mixes 1, 2, 3, 5 and 7 are shown in Figure 8 and indicate that R.Block Hg alone (B1 in Figure 8) as both network blocker and surface blocker is as effective at blocking as Salmon gDNA network block combined with Cot-IDNA, and more effective than Cot-IDNA blocker alone when performing in solution target capture, and that a combination of R.Block -Hg and R.Block -Sg as hybridisation and surface blocker (B1/B2 in Figure 8) is more effective than Cot-1 DNA or a mix of Cot-1 DNA and salmon gDNA mixes.
The combination of network blocking with R.Block -Hg and surface blocking with R.Block - Sg was in fact approximately 4 times as effective as using Cot-1 DNA blocker alone and approximately 2 times as effective as using Cot-1 DNA and salmon DNA (as network and surface blockers respectively), as shown in Figure 8.
In addition, several potential advantages of the R.Block have been identified over the use of DNA based blockers. For example R.Block -Hc, -Sg and -Hg not only block surface interactions, but also mask interspersed repetitive sequences. This is beneficial when performing in solution target capture.
It was also found that R.Block-Hg was at least twice as effective a network blocker as Cot-1 DNA. R.Block-Sg and Salmon gDNA worked with similar efficiencies when used as surface blockers. Cot-1 DNA did not perform particularly well as a surface blocker or a network blocker. The best combination of blockers was determined to be R.Block-Hg as the network blocker with either R.Block-Sg or Salmon gDNA as the surface blocker.
The above examples and embodiments are described by way of example only. Many variations are possible without departing from the scope of the invention as defined in the appended claims.

**Claims**

1.  A method of nucleic acid sequence hybridisation comprising the steps of:

    a) hybridising sample derived DNA that includes region of interest sequences with probe nucleic acid sequences; and
    b) blocking or masking repetitive DNA sequences in the DNA by mixing the sample derived DNA that includes region of interest sequences with ribonucleic acid molecules comprising the same or substantially similar repetitive sequences as those of the sample derived nucleic acids during step a), wherein the mixing is carried out at between 50 °C and 80 °C.

2.  A method as claimed in claim 1 wherein the ribonucleic acid molecules comprise an RNA transcription product, preferably transcribed from genomic DNA sequences from the same species as the sample being processed.

3.  A method as claimed in claim 2 wherein the RNA transcription product comprises a transcription product of one or more fragments of human genomic DNA, human Cot-1 DNA or salmon DNA.

4.  A method as claimed in any one of claims 1 to 3, comprising adding a mixture of two or more ribonucleic acid molecules.

5.  A method as claimed in claim 4 wherein the mixture comprises a transcription product of mammalian genomic DNA and a transcription product of a DNA selected from fish, bird, reptile, amphibian, plant or fungus.

6.  A method as claimed in claim 5 wherein the mixture comprises an RNA transcription product of human genomic DNA and an RNA transcription product of Cot-1 DNA or of salmon DNA.

**7.** A method as claimed in any preceding claim comprising adding a mixture of at least one ribonucleic acid and at least one deoxyribonucleic acid as masking or blocking agents.

**8.** A method as claimed in claim 7 wherein the method comprises mixing an RNA transcription product of a DNA molecule and a Cot-1 DNA or salmon genomic DNA molecule with at least one sample nucleic acid.

**9.** A method as claimed in any preceding claim wherein the method further comprises eliminating the ribonucleic acid molecule from repetitive DNA sequences using a ribonuclease (RNase).

**Patentansprüche**

**1.** Verfahren zur Nukleinsäuresequenzhybridisierung aufweisend die Schritte:

a) Hybridisieren von einer Probe abgeleiteten DNA, welche zu untersuchende Sequenzenbereiche enthält, mit Probennukleinsequenzen;
b) Blockieren oder Maskieren von sich wiederholenden DNA Sequenzen in der DNA durch Mischen der von der Probe abgeleiteten DNA, welche zu untersuchende Sequenzenbereiche enthält mit Ribonukleinsäuremolekülen, welche dieselben oder im Wesentlichen gleiche sich wiederholende Sequenzen aufweisen, wie die von der Probe abgeleiteten Nukleinsäuren während Schritt a), wobei das Mischen zwischen 50°C und 80°C durchgeführt wird.

**2.** Verfahren nach Anspruch 1, wobei die Ribonukleinsäuremoleküle ein RNA Transkriptionsprodukt aufweisen, vorzugsweise transkribiert aus genomischen DNA Sequenzen von derselben Spezies wie die zu verarbeitende Probe.

**3.** Verfahren nach Anspruch 2, wobei das RNA Transkriptionsprodukt ein Transkriptionsprodukt von einem oder mehreren Fragmenten von genomischen humanoiden DNA, humanoiden Cot-1-DNA oder Lachs-DNA aufweist.

**4.** Verfahren nach einem der Ansprüche 1 bis 3, aufweisend das Hinzufügen einer Mischung von zwei oder mehr Ribonukleinsäuremolekülen.

**5.** Verfahren nach Anspruch 4, wobei die Mischung ein Transkriptionsprodukt von genomischer Säugetier-DNA und ein Transkriptionsprodukt von einer DNA ausgewählt aus Fischen, Vögeln, Reptilien, Amphibien, Pflanzen oder Pilzen, aufweist.

**6.** Verfahren nach Anspruch 5, wobei die Mischung ein RNA Transkriptionsprodukt aus menschlicher genomischer DNA und einem RNA Transkriptionsprodukt aus Cot-1 -DNA oder von Lachs-DNA aufweist.

**7.** Verfahren nach einem der vorhergehenden Ansprüche, aufweisend das Hinzufügen einer Mischung von mindestens einer Ribonukleinsäure und mindestens einer Desoxyribonukleinsäure als Maskierungs- oder Blockierungsmittel.

**8.** Verfahren nach Anspruch 7, wobei das Verfahren aufweist das Mischen eines RNA Transkriptionsproduktes eines DNA-Moleküls und eines Cot.1-DNA- oder genomischen Lachs-DNA-Moleküls mit mindestens einer Probe Nukleinsäure.

**9.** Verfahren nach einem der vorhergehenden Ansprüche, wobei das Verfahren des Weiteren aufweist das Eliminieren des Ribonukleinsäuremoleküls von sich wiederholenden DNA Frequenzen unter Verwendung einer Ribonuklease (RNase).

**Revendications**

**1.** Procédé d'hybridation de séquence d'acides nucléiques comprenant les étapes consistant à :

a) hybrider un ADN dérivé d'un échantillon qui inclut des séquences de région d'intérêt avec des séquences d'acides nucléiques sondes ; et
b) bloquer ou masquer des séquences d'ADN répétitives dans l'ADN en mélangeant l'ADN dérivé de l'échantillon qui inclut des séquences de région d'intérêt avec des molécules d'acide ribonucléique comprenant les mêmes séquences répétitives ou des séquences répétitives sensiblement similaires à celles des acides nucléiques dérivés de l'échantillon durant l'étape a), dans lequel le mélange est réalisé entre 50 °C et 80 °C.

2. Procédé selon la revendication 1 dans lequel les molécules d'acide ribonucléique comprennent un produit de transcription d'ARN, de préférence transcrit à partir de séquences d'ADN génomique issues de la même espèce que l'échantillon en cours de traitement.

3. Procédé selon la revendication 2 dans lequel le produit de transcription d'ARN comprend un produit de transcription d'un ou de plusieurs fragments d'ADN génomique humain, d'ADN Cot-1 humain ou d'ADN de saumon.

4. Procédé selon l'une quelconque des revendications 1 à 3, comprenant l'ajout d'un mélange de deux ou plus molécules d'acide ribonucléique.

5. Procédé selon la revendication 4 dans lequel le mélange comprend un produit de transcription d'ADN génomique mammifère et un produit de transcription d'un ADN sélectionné parmi un poisson, un oiseau, un reptile, un amphibien, une plante ou un champignon.

6. Procédé selon la revendication 5 dans lequel le mélange comprend un produit de transcription d'ARN d'ADN génomique humain et un produit de transcription d'ARN d'ADN Cot-1 ou d'ADN de saumon.

7. Procédé selon l'une quelconque des revendications précédentes comprenant l'ajout d'un mélange d'au moins un acide ribonucléique et d'au moins un acide désoxyribonucléique comme agents de masquage ou de blocage.

8. Procédé selon la revendication 7 dans lequel le procédé comprend le mélange d'un produit de transcription d'ARN d'une molécule d'ADN et d'une molécule d'ADN Cot-1 ou d'ADN génomique de saumon avec au moins un acide nucléique d'échantillon.

9. Procédé selon l'une quelconque des revendications précédentes dans lequel le procédé comprend en outre l'élimination de la molécule d'acide ribonucléique de séquences d'ADN répétitives à l'aide d'une ribonucléase (RNase).

Figure 1

Figure 2

Figure 3

Figure 4

Figure 5

| R.Block | Enrichment power (fr/ft): Target only | Enrichment power (fr/ft): Target plus 100bp of sequence on each side |
|---|---|---|
| No blocker q | 358 | NA |
| 10μg h.gDNA | 2138 | 2299 |
| 5μg h.gDNA | 1943 | 2070 |
| 10μg h.Cot-1 | 1840 | 1961 |
| 5μg h.Cot-1 | 1427 | 1496 |

Figure 6

Figure 7

Figure 8

EP 3 303 615 B1